# FASCICULE DE BREVET EUROPEEN

(11) **EP 4 277 593 B1**
(45) Date de publication et mention de la délivrance du brevet: **09.04.2025**
(21) Numéro de dépôt: 22700143.5
(22) Date de dépôt: 12.01.2022
(51) Int. Cl.: A61K 8/60, A61Q 19/00, A61K 8/73

(54) **METHODE COSMETIQUE ET COMPOSITION A BASE DE FRUCTOOLIGOSACCHARIDES A CHAINE COURTE ET D'UN AMIDON NATIF**
KOSMETISCHES VERFAHREN UND ZUSAMMENSETZUNG AUF BASIS VON KURZKETTIGEN FRUCTOOLIGOSACCHARIDEN UND EINER NATIVEN STÄRKE
COSMETIC METHOD AND COMPOSITION BASED ON SHORT-CHAIN FRUCTOOLIGOSACCHARIDES AND A NATIVE STARCH

(30) Priorité: 12.01.2021 FR 2100251; 18.11.2021 FR 2112189
(43) Date de publication de la demande: 22.11.2023
(73) Titulaire: Beghin, Meiji, 77230 Moussy-le-Vieux (FR); Tereos Starch & Sweeteners Europe, 77230 Moussy-le-Vieux (FR)
(72) Inventeur: LE BOURGOT, Cindy, 77230 MOUSSY-LE-VIEUX (FR); METZGER, Nicolas, 77230 MOUSSY-LE-VIEUX (FR); MEUNIER, Claire, 77230 MOUSSY-LE-VIEUX (FR); SY, Perrine, 77230 MOUSSY-LE-VIEUX (FR)
(74) Mandataire: Icosa
(86) Numéro de dépôt international: PCT/EP2022/050566
(87) Numéro de publication internationale: WO 2022/152762

(56) Documents cités:
- WO-A1-2021/023695
- CN-A- 103 315 921
- CN-A- 103 622 901
- FR-A1- 2 831 059
- FR-A1- 2 874 825
- FR-A1- 2 916 634
- FR-A1- 2 954 140
- FR-A1- 3 097 436
- FR-A1- 3 097 441
- FR-B1- 2 831 059
- US-A- 5 518 733

## Description

### DOMAINE DE L'INVENTION

La présente invention se situe dans le domaine de la cosmétique. Plus précisément, elle se situe dans le domaine du maintien de l'équilibre du microbiote cutané.

### ÉTAT DE LA TECHNIQUE

La peau est un écosystème complexe et dynamique représentant le plus grand organe du corps humain. Outre la barrière physique, le microbiote cutané garantit une protection et une barrière biologique en rivalisant avec les agents pathogènes et en communiquant étroitement avec les cellules et les composants du système immunitaire. Ainsi, le microbiote cutané peut être considéré comme un acteur essentiel pour le maintien d'une peau saine (1). Le microbiote cutané comprend des microorganismes résidents qui se trouvent couramment dans la peau, considérés comme commensaux, ce qui signifie qu'ils sont généralement inoffensifs et offrent très probablement certains avantages à l'hôte (2, 3). Parmi les espèces résidentes du microbiote cutané, il y a des bactéries propioniques comme *Propionibacterium acnes,* également appelée *Cutibacterium acnes,* des staphylocoques à coagulase négative comme *Staphylococcus epidermidis* et d'autres types comme *Corynebacteria, Micrococci et Acinebacter. S. epidermidis* est une bactérie commensale, faisant partie du microbiote cutané que l'on peut qualifier de « normal », qui sert à protéger la peau humaine des infections et autres agressions environnementales (4). *C. acnes* a été liée à l'état cutané de l'acné et associée à de nombreuses pathologies cutanées (5, 6). Le microbiote cutané comprend également des microorganismes transitoires, dont les espèces les plus courantes sont *Staphylococcus aureus, Escherichia coli* et *Pseudomonas aeruginosa* (4, 7). S. *aureus* a été identifiée comme un pathogène important lors de la sur-colonisation de la peau et est impliquée dans le développement de maladies de la peau humaine (2). La composition et l'abondance du microbiote cutané varient considérablement selon les parties du corps, entre les individus et au cours du temps, ce qui se traduit par une communauté bactérienne extrêmement dynamique et très fluctuante (8, 9). CN 103 622 901 A, CN 103 315 921 A, FR 2 874 825 A1, US 5 518 733 A, FR 2 954 140 A1, FR 2 916 634 A1, FR 3 097 436 A1 et FR 3 097 441 A1 divulguent des compositions comprenant des fructo-oligosaccharides à chaîne courte pour équilibrer le microbiote cutané.

Le microbiote cutané humain est récemment devenu un centre d'intérêt pour les domaines dermatologique et cosmétique. Comprendre le microbiote cutané et comment maintenir son équilibre délicat sont des étapes essentielles pour appréhender les mécanismes responsables d'une peau saine et de son apparence. Les déséquilibres dans la composition du microbiote cutané, également appelés dysbioses, sont associés à plusieurs pathologies cutanées comme l'acné, l'eczéma ou les allergies, ainsi qu'à des conditions non pathologiques telles que la peau sensible, l'irritation ou la peau sèche.

Par conséquent, le développement d'approches préservant ou rétablissant l'équilibre du microbiote représente une nouvelle cible pour favoriser la santé de la peau. Il existe donc des besoins pour une méthode simple et efficace permettant d'équilibrer le microbiote de la peau.

Dans ce cadre, la Demanderesse a démontré que l'utilisation cosmétique d'une composition cosmétique comprenant au moins un fructo-oligosaccharide à chaîne courte permet d'équilibrer le microbiote cutané de façon sélective, en stimulant les organismes bénéfiques et en limitant les organismes non bénéfiques. Cette utilisation cosmétique permet d'améliorer l'apparence et/ou le confort de la peau, des phanères et/ou des muqueuses.

### RÉSUMÉ

Ainsi, la présente invention concerne une méthode cosmétique pour équilibrer le microbiote cutané, comprenant une étape d'application sur tout ou partie de la peau, des phanères et/ou des muqueuses d'une composition cosmétique comprenant, dans un support cosmétiquement acceptable, au moins un fructo-oligosaccharide à chaîne courte et au moins un amidon natif.

Dans un mode de réalisation, la méthode cosmétique selon l'invention est pour améliorer l'apparence et/ou le confort cutané.

Dans un mode de réalisation de l'invention, améliorer l'apparence et/ou le confort cutané comprend réduire au moins un désagrément cutané choisi dans le groupe constitué par les rougeurs, les tiraillements, la peau terne et les démangeaisons.

Dans un mode de réalisation, le au moins un fructo-oligosaccharide est issu de la betterave sucrière.

Dans un mode de réalisation, le au moins un fructo-oligosaccharide à chaîne courte est un mélange :
- d'un fructo-oligosaccharide contenant une unité de glucose et deux unités de fructose, aussi appelé 1-kestose ou GF2, présentant un degré de polymérisation égal à 3;
- d'un fructo-oligosaccharide contenant une unité de glucose et trois unités de fructose, aussi appelé nystose ou GF3, présentant un degré de polymérisation égal à 4 ; et
- d'un fructo-oligosaccharide contenant une unité de glucose et quatre unités de fructose, aussi appelé 1-bêta-fructofuranosylnystose ou GF4, présentant un degré de polymérisation égal à 5.

Dans un mode de réalisation, le au moins un amidon natif est au moins un amidon natif de maïs.

Dans un mode de réalisation, le au moins un amidon natif est compris dans la composition cosmétique en une quantité de 0,5 à 20% en poids par rapport au poids total de la composition cosmétique et/ou le au moins un fructo-oligosaccharide à chaîne courte est compris dans la composition cosmétique en une quantité d'au moins 0,5% en poids par rapport au poids total de la composition cosmétique.

Dans un mode de réalisation de l'invention, équilibrer le microbiote cutané comprend favoriser le développement et/ou la croissance de *Staphylococcus epidermidis,*

et/ou limiter le développement et/ou la croissance d'au moins un organisme parmi *Cutibacterium acnes* et *Staphylococcus aureus.*

Dans un mode de réalisation, la méthode cosmétique selon l'invention est mise en œuvre après un événement dysbiotique.

La présente invention concerne également l'utilisation non-thérapeutique d'une composition cosmétique comprenant, dans un support cosmétiquement acceptable, au moins un fructo-oligosaccharide à chaîne courte, et au moins un amidon natif, pour équilibrer le microbiote cutané.

La présente invention concerne également une composition cosmétique comprenant, dans un support cosmétiquement acceptable, au moins un fructo-oligosaccharide à chaîne courte et au moins un amidon natif, dans laquelle le au moins un fructo-oligosaccharide à chaîne courte est un mélange :
- d'un fructo-oligosaccharide contenant une unité de glucose et deux unités de fructose, aussi appelé 1-kestose ou GF2, présentant un degré de polymérisation égal à 3;
- d'un fructo-oligosaccharide contenant une unité de glucose et trois unités de fructose, aussi appelé nystose ou GF3, présentant un degré de polymérisation égal à 4 ; et
- d'un fructo-oligosaccharide contenant une unité de glucose et quatre unités de fructose, aussi appelé 1-bêta-fructofuranosylnystose ou GF4, présentant un degré de polymérisation égal à 5.

Dans un mode de réalisation, le au moins un amidon natif est un amidon natif de maïs.

Selon un mode de réalisation, le au moins un amidon natif est compris en une quantité de 0,5 à 20% en poids ou de de plus de 5% à 20% en poids par rapport au poids total de la composition cosmétique. Selon un mode de réalisation, le au moins un fructo-oligosaccharide à chaîne courte est compris en une quantité d'au moins 0,5% en poids par rapport au poids total de la composition cosmétique.

Dans un mode de réalisation, la composition cosmétique est sous la forme d'un produit dermo-cosmétique, d'un produit capillaire, d'un produit d'hygiène intime, d'un pansement et/ou d'un produit cicatrisant.

### DÉFINITIONS

Dans la présente invention, les termes ci-dessous sont définis de la manière suivante :
Par « **améliorer l'apparence et/ou le confort de la peau, des phanères et/ou des muqueuses** », on désigne diminuer, voire supprimer, le nombre, l'étendue, l'occurrence et/ou l'intensité d'au moins un désagrément de la peau, des phanères et/ou des muqueuses, dit désagrément cutané. Le désagrément cutané peut notamment être choisi dans le groupe constitué par une rougeur, un tiraillement, une peau sèche, une peau sensible, une démangeaison, une irritation et une peau terne.
Par « **amidon** », on désigne un polysaccharide composé de chaînes de molécules de D-glucose, les unités de glucose étant liées pour environ 95% par des liaisons alpha-1,4 et pour environ 5% par des liaisons alpha-1,6. Les deux homopolymères constitutifs de l'amidon sont l'amylose (qui résulte de l'enchaînement linéaire d'unités glucose liées par des liaisons alpha-1,4) et l'amylopectine (qui présente une structure ramifiée dans laquelle des fragments linéaires de type amylose sont liés par des liaisons alpha-1,6) ; du fait de la présence de l'amylopectine, l'amidon est partiellement ramifié.
Par « **amidon natif** », on désigne un amidon qui n'a subi aucune modification après extraction du végétal.
Par « **compris entre X et** Y », on désigne la gamme de valeurs comprises entre X et Y, les bornes X et Y étant incluses dans ladite gamme.
Par « environ » une valeur nominale, on désigne dans la présente invention un intervalle compris entre plus et moins 10% de la valeur nominale, de préférence entre plus et moins 5% de la valeur nominale, en particulier entre plus et moins 1% de la valeur nominale.
Par « **équilibrer le microbiote cutané** », on désigne l'action de maintenir, préserver et/ou rétablir l'équilibre du microbiote cutané. Equilibrer le microbiote cutané comprend favoriser le développement et/ou la croissance d'au moins un organisme considéré comme bénéfique à la peau, aux phanères et/ou aux muqueuses, et/ou limiter le développement et/ou la croissance d'au moins un organisme considéré comme non bénéfique à la peau, aux phanères et/ou aux muqueuses, et/ou rétablir la diversité des organismes à la surface de la peau, des phanères et/ou des muqueuses. Dans certains modes de réalisation, équilibrer le microbiote cutané comprend favoriser le développement et/ou la croissance de *Staphylococcus epidermidis,* et/ou limiter le développement et/ou la croissance d'au moins un organisme parmi *Cutibacterium acnes* et *Staphylococcus aureus.* Dans un mode de réalisation particulier, équilibrer le microbiote cutané comprend favoriser le développement et/ou la croissance de *Staphylococcus epidermidis,* et limiter le développement et/ou la croissance de *Cutibacterium acnes* et *Staphylococcus aureus.*
Par « **évènement dysbiotique » ou « dysbiose** », on désigne un évènement au cours duquel l'équilibre du microbiote cutané est altéré, soit par croissance et/ou développement d'au moins un organisme considéré comme non bénéfique à la peau, aux phanères et/ou aux muqueuses, soit par diminution et/ou disparition d'au moins un organisme considéré comme bénéfique à la peau, aux phanères et/ou aux muqueuses. La dysbiose peut être à l'origine de nombreux désagréments et de pathologies cutanés. En cas de dysbiose, les réponses immunitaires de la peau, des phanères et/ou des muqueuses peuvent être modifiées et certaines espèces bactériennes pathogènes peuvent se développer davantage.

Parmi les évènements dysbiotiques, on peut citer notamment ceux consécutifs à des pathologies cutanées telles que l'acné, le psoriasis, l'hidradénite suppurée ou maladie de Verneuil et la dermatite atopique. Dans certains modes de réalisation, l'évènement dysbiotique est l'acné et le déséquilibre du microbiote comprend le développement d'une souche de *Propionibacterium acnes.* Dans certains modes de réalisation, l'évènement dysbiotique est le psoriasis et le déséquilibre du microbiote comprend le développement de bactéries considérées comme non bénéfiques à la peau. Dans certains modes de réalisation, l'évènement dysbiotique est l'hidradénite suppurée ou maladie de Verneuil et le déséquilibre du microbiote comprend le développement de bactéries et une diversité des bactéries plus importante que dans le microbiote normal. Dans certains modes de réalisation, l'évènement dysbiotique est la dermatite atopique et le déséquilibre du microbiote comprend le développement de *Staphylococcus aureus* et une diversité des bactéries moins importante que dans le microbiote normal.

Dans certains modes de réalisation, l'évènement dysbiotique est l'occurrence d'une peau sensible, d'une irritation, d'une démangeaison, d'une rougeur, d'un tiraillement et/ou d'une sécheresse cutanée. De préférence, l'occurrence d'une peau sensible, d'une irritation, d'une démangeaison, d'une rougeur, d'un tiraillement et/ou d'une sécheresse cutanée n'est pas pathologique ni associée à une pathologie cutanée.

Par « **Fructo-oligosaccharides** » ou « **FOS** », on désigne des oligomères ayant un degré de polymérisation maximal de 10, composés de D-fructoses liés par des liaisons beta-1,2 et pouvant contenir une molécule de glucose à l'une des extrémités. Les FOS font partie des fructanes. Ils sont présents dans la nature dans un certain nombre de plantes comme l'oignon, la chicorée, l'artichaut, l'ail, la banane, l'asperge et la betterave. Ils peuvent aussi être produits industriellement par deux procédés différents : par hydrolyse partielle de l'inuline de chicorée par une endoinulinase spécifique (l'inuline étant un polymère linéaire de fructose lié en beta-2,1 avec à l'une des extrémités un glucose lié en alphal-beta2) ou par synthèse enzymatique.

Par « **Fructo-oligosaccharides à chaîne courte** » ou « **scFOS** », on désigne des fructo-oligosaccharides constitués de 2 à 4 molécules de fructose et contenant une molécule de glucose à l'une des extrémités. Dans un mode de réalisation, les fructo-oligosaccharides à chaîne courte sont issus de la betterave à sucre, du blé, de l'orge, du seigle, de la canne, de l'asperge, de la banane, de l'artichaut, de l'ail et/ou de l'oignon. Dans un mode de réalisation, les fructo-oligosaccharides sont issus de la betterave à sucre. De préférence, il s'agit des fructo-oligosaccharides commercialisés sous la dénomination commerciale Actilight^{®} ou FOSbeauty^{®}.

Par **« GF2** », **« GF3 »** et **« GF4** », on désigne les dénominations données respectivement au 1-kestose, au nystose et au 1-bêta-fructofuranosylnystose. L'initiale G correspond à une unité de glucose et l'initiale F correspond à une unité de fructose.

Par « **microbiote de la peau » ou « microbiote cutané** », on désigne la communauté de microorganismes opportunistes, saprobiontes, souvent symbiotes et parfois pathogènes qui composent la flore présente à la surface de la peau, des phanères et/ou des muqueuses. De préférence, le microbiote cutané est le microbiote présent à la surface de la peau et/ou des phanères. Dans un mode de réalisation, le microbiote cutané est le microbiote présent à la surface de la peau. Dans un autre mode de réalisation, le microbiote cutané est le microbiote présent à la surface des phanères. Dans un dernier mode de réalisation, le microbiote cutané est le microbiote présent à la surface des muqueuses. De préférence, le microbiote cutané est le microbiote cutané humain.

Par « **phanères** », on désigne les productions tégumentaires issues de l'ectoderme et caractérisées par un taux élevé de kératinisation. Dans un mode de réalisation, les phanères sont les cheveux, les poils et/ou les ongles. De préférence, les phanères sont les cheveux.

Par « **support cosmétiquement acceptable** », on désigne tout adjuvant ou excipient permettant la fabrication, la conservation et/ou l'administration de la composition cosmétique. De préférence, il s'agit d'un milieu qui est compatible avec la peau, les phanères et/ou les muqueuses, ayant une couleur, une odeur et un ressenti plaisants, et ne causant aucun inconfort inacceptable (picotements, tiraillements ou rougeurs notamment) susceptible de décourager le consommateur d'utiliser la composition cosmétique.

### DESCRIPTION DÉTAILLÉE

### Méthode cosmétique

La présente invention porte sur une méthode cosmétique pour équilibrer le microbiote cutané. Equilibrer le microbiote cutané permet notamment d'améliorer l'apparence et/ou le confort cutané.

La méthode cosmétique selon l'invention comprend l'application sur la peau, ses phanères et/ou les muqueuses d'au moins un fructo-oligosaccharide à chaîne courte. En particulier, le au moins un fructo-oligosaccharide à chaîne courte est compris dans une composition cosmétique comprenant en outre un support cosmétiquement acceptable.

La méthode cosmétique selon l'invention permet d'améliorer l'esthétique et/ou le confort de la peau, des phanères et/ou des muqueuses sur laquelle ou lesquelles la composition est appliquée. Elle peut permettre notamment la diminution de désagréments tels que rougeurs, tiraillements, peau terne et/ou démangeaisons. Les effets obtenus sont uniquement en surface de la peau, des phanères et/ou des muqueuses, et sont uniquement cosmétiques et non thérapeutiques. Ainsi, la méthode cosmétique selon l'invention est une méthode non thérapeutique en ce qu'elle ne permet pas de traiter ou diminuer les symptômes d'une pathologie, mais uniquement d'améliorer l'apparence et/ou le confort de la peau, des phanères et/ou des muqueuses en contribuant à maintenir, préserver et/ou rétablir l'équilibre du microbiote cutané.

De préférence, l'application est une application sur la peau et/ou les phanères, ou application topique.

L'application de la composition cosmétique peut être réalisée par toute méthode adaptée connue dans l'art. L'application peut être réalisée au moyen d'un applicateur, tel qu'une mousse, un embout floqué ou non, un feutre, une brosse, un peigne, un pinceau, un tissé, ou un non-tissé. Alternativement, la composition cosmétique peut être appliquée dans la mise en œuvre de la méthode selon l'invention sans applicateur, notamment avec un ou plusieurs doigts.

L'application peut être mise en œuvre pendant toute durée et à toute fréquence adaptées. La durée et/ou la fréquence d'application peuvent notamment être adaptées en fonction de la zone de peau, de phanères et/ou de muqueuse concernée, de la surface de la peau, des phanères et/ou des muqueuses concernée, de l'apparence de la peau, des phanères et/ou des muqueuses, et/ou de l'état du microbiote cutané, notamment de son déséquilibre, avant l'application de la composition cosmétique.

La composition cosmétique peut être appliquée pendant toute durée suffisante pour maintenir, restaurer et/ou équilibrer le microbiote cutané. La composition cosmétique peut être appliquée pendant toute durée suffisante pour améliorer l'apparence et/ou le confort de la peau, des phanères et/ou des muqueuses. L'application peut ainsi être réalisée pendant une durée comprise entre 1 jour et 6 mois, de préférence entre 2 jours et 1 mois, en particulier entre 7 jours et 21 jours.

La fréquence d'application de la composition cosmétique dans la méthode selon l'invention peut être toute fréquence suffisante pour maintenir, restaurer et/ou équilibrer le microbiote cutané. La fréquence d'application de la composition cosmétique dans la méthode selon l'invention peut être toute fréquence suffisante pour améliorer l'apparence et/ou le confort de la peau, des phanères et/ou des muqueuses. L'application peut ainsi être mise en œuvre trois fois par jour, deux fois par jour, une fois par jour, une fois tous les deux jours ou une fois par semaine par exemple.

Dans des modes de réalisation, l'application dans la méthode de l'invention peut être une application unique, par exemple suite à l'apparition d'au moins un signe choisi par exemple dans le groupe constitué par une rougeur, un tiraillement, une peau sèche, une peau sensible, une démangeaison, une irritation et une peau terne.

Dans la méthode selon l'invention, l'application de la composition cosmétique peut être réalisée à tout moment de la journée. Dans un mode de réalisation, l'application est réalisée le matin. Dans un mode de réalisation, l'application est réalisée le soir avant le coucher.

L'invention a également pour objet l'utilisation dans une composition cosmétique d'une quantité efficace d'au moins un fructo-oligosaccharide à chaîne courte, la composition étant destinée à équilibrer le microbiote cutané.

L'invention a également pour objet l'utilisation non-thérapeutique d'une composition cosmétique comprenant, dans un support cosmétiquement acceptable, au moins un fructo-oligosaccharide à chaîne courte pour équilibrer le microbiote cutané.

### Composition cosmétique

La présente invention concerne également une composition cosmétique comprenant au moins un fructo-oligosaccharide à chaîne courte et au moins un support cosmétiquement acceptable.

Le au moins un fructo-oligosaccharide à chaîne courte peut être compris dans la composition cosmétique selon l'invention en toute quantité adaptée. En particulier, il peut être compris dans la composition cosmétique en une quantité efficace pour améliorer l'apparence et/ou le confort cutané en contribuant à restaurer, maintenir et/ou préserver l'équilibre du microbiote cutané.

Dans un mode de réalisation, le au moins un fructo-oligosaccharide à chaîne courte est compris dans la composition cosmétique en une quantité de 0,1% à 50% en poids par rapport au volume total de la composition cosmétique. Dans un mode de réalisation, le au moins un fructo-oligosaccharide à chaîne courte est compris dans la composition cosmétique en une quantité de 1 à 5% en poids par rapport au volume total de la composition cosmétique. Dans un mode de réalisation, le au moins un fructo-oligosaccharide à chaîne courte est compris dans la composition cosmétique en une quantité de 5 à 50%, de préférence de 5 à 20%, en particulier de 5 à 15% en poids par rapport au volume total de la composition cosmétique. Dans un mode de réalisation, le au moins un fructo-oligosaccharide à chaîne courte est compris dans la composition cosmétique en une quantité strictement inférieure à 2,5% en poids par rapport au volume total de la composition cosmétique. Dans un mode de réalisation, le au moins un fructo-oligosaccharide à chaîne courte est compris dans la composition cosmétique en une quantité strictement supérieure à 2,5% en poids par rapport au volume total de la composition cosmétique. Dans un mode de réalisation, le au moins un fructo-oligosaccharide à chaîne courte est compris dans la composition cosmétique en une quantité d'environ 5% en poids par rapport au volume total de la composition cosmétique.

Dans un mode de réalisation, le au moins un fructo-oligosaccharide à chaîne courte est compris dans la composition cosmétique en une quantité d'au moins 0,5% en poids par rapport au poids total de la composition cosmétique. Dans un mode de réalisation, le au moins un fructo-oligosaccharide à chaîne courte est compris dans la composition cosmétique en une quantité de 0,5 à 5% en poids par rapport au poids total de la composition cosmétique. Dans un mode de réalisation, le au moins un fructo-oligosaccharide à chaîne courte est compris dans la composition cosmétique en une quantité d'environ 0,5% en poids par rapport au poids total de la composition cosmétique.

Dans un mode de réalisation préféré, le au moins un fructo-oligosaccharide à chaîne courte est compris dans la composition cosmétique en une quantité d'environ 1% en poids par rapport au poids total de la composition cosmétique.

Dans un mode de réalisation, le au moins un fructo-oligosaccharide à chaîne courte est compris dans la composition cosmétique en une quantité d'environ 2,5% en poids par rapport au poids total de la composition cosmétique.

Dans un mode de réalisation, le au moins un fructo-oligosaccharide à chaîne courte est compris dans la composition cosmétique en une quantité d'environ 5,0% en poids par rapport au poids total de la composition cosmétique.

Dans un mode de réalisation, le au moins un fructo-oligosaccharide à chaîne courte est un mélange d'au moins deux, de préférence au moins trois, fructo-oligosaccharides à chaîne courte.

Le au moins un fructo-oligosaccharide à chaîne court dans la composition cosmétique selon l'invention est un mélange :
- d'un fructo-oligosaccharide contenant une unité de glucose et deux unités de fructose, aussi appelé 1-kestose ou GF2, présentant un degré de polymérisation égal à 3 ;
- d'un fructo-oligosaccharide contenant une unité de glucose et trois unités de fructose, aussi appelé nystose ou GF3, présentant un degré de polymérisation égal à 4 ; et
- d'un fructo-oligosaccharide contenant une unité de glucose et quatre unités de fructose, aussi appelé 1-bêta-fructofuranosylnystose ou GF4, présentant un degré de polymérisation égal à 5.

Selon une caractéristique préférée, les unités de fructose des fructo-oligosaccharides GF2, GF3 et GF4 sont liées entre elles par des liaisons bêta(2-1) et l'unité de glucose terminale est liée à une unité de fructose par une liaison alphal-bêta2.

Selon un mode de réalisation, le au moins un fructo-oligosaccharide à chaîne courte est obtenu par une réaction enzymatique, de préférence faisant intervenir une bêta-fructofuranosidase sur du saccharose. Selon une caractéristique avantageuse, ledit saccharose est issu de la betterave sucrière. A partir des molécules de saccharose, le produit obtenu à l'issue de la réaction enzymatique est un mélange de saccharose, glucose et de fructo-oligosaccharides à courte chaîne. Avantageusement, ce produit est purifié par chromatographie.

Selon un mode de réalisation, le au moins un fructo-oligosaccharide à chaîne courte comprend entre 30 et 45% de GF2, entre 40 et 60% de GF3, entre 5 et 15% de GF4 et entre 1 et 10% de mono- et disaccharides (glucose et saccharose), préférentiellement entre 35 et 40% de GF2, entre 40 et 55% de GF3, entre 7 et 12% de GF4 et entre 3 et 8% de mono- et disaccharides (glucose et saccharose), plus préférentiellement environ 37% de GF2, environ 53% de GF3, environ 10% de GF4 et moins de 7% de mono- et disaccharides (glucose et saccharose), les pourcentages étant exprimés en poids par rapport au poids total du mélange de fructo-oligosaccharides.

Selon un mode de réalisation préféré, le au moins un fructo-oligosaccharide à chaîne courte est le produit commercialisé sous le nom de marque ACTILIGHT^{®} par l'entreprise TEREOS^{®}. Plus préférentiellement, le au moins un fructo-oligosaccharide à chaîne courte est le produit commercialisé sous le nom de marque ACTILIGHT^{®} 950P, ACTILIGHT^{®} 950S ou ACTILIGHT^{®} 951S par l'entreprise TEREOS^{®}. Selon un deuxième mode de réalisation préféré, le au moins un fructo-oligosaccharide à chaîne courte est le produit commercialisé sous le nom de marque FOSbeauty^{®} par l'entreprise TEREOS^{®}.

La composition cosmétique selon l'invention peut se présenter sous toutes les formes normalement utilisées pour une application topique, notamment sous forme hydroalcoolique, d'une émulsion huile-dans-eau ou eau-dans-huile ou multiple, d'un gel huileux, ou d'un produit anhydre liquide, pâteux ou solide ou sous forme de dispersion en présence de sphérules. Ces compositions sont préparées selon les méthodes usuelles.

La composition cosmétique peut se présenter sous toutes les formes galéniques envisageables. La composition peut avoir la forme d'une solution aqueuse, alcoolique, hydroalcoolique ou huileuse, d'une dispersion du type lotion ou sérum, d'une suspension, de microcapsules ou microparticules ; de dispersions vésiculaires de type ionique et/ou non ionique, d'une lotion aqueuse, huileuse ou sous forme de sérum ; d'une mousse, d'une préparation solide, par exemple de stick ; d'une composition pour aérosol comprenant également un agent propulseur sous pression, d'un gel, ou sous forme de patch.

Cette composition peut être plus ou moins fluide et avoir l'aspect d'une crème blanche ou colorée, d'une pommade, d'un lait, ou d'une pâte. Elle peut éventuellement être appliquée sur la peau, ses phanères et/ou les muqueuses sous forme d'aérosol.

La composition cosmétique selon l'invention peut notamment être sous la forme d'un produit dermo-cosmétique, d'un produit capillaire, d'un produit d'hygiène intime, d'un pansement et/ou d'un produit cicatrisant. Dans un mode de réalisation, la composition cosmétique selon l'invention est sous la forme d'un produit dermo-cosmétique, d'un produit capillaire, d'un produit d'hygiène intime et/ou d'un pansement. Dans un mode de réalisation, la composition cosmétique selon l'invention est sous la forme d'un produit dermo-cosmétique, d'un produit capillaire et/ou d'un produit d'hygiène intime.

La composition selon l'invention peut se présenter sous la forme d'une composition de nettoyage, de protection ou de soin pour le visage, ou pour le corps (par exemple crème de jour, crème de nuit, crème démaquillante, laits corporels de protection ou de soins tels que crème hydratante ou crème solaire, lotion, gel ou mousse pour le soin de la peau), une composition de maquillage telle que le fond de teint.

La composition selon l'invention peut se présenter sous la forme d'un produit capillaire tel qu'un shampooing, notamment un shampooing solide, un après-shampoing, un produit de coloration, un masque capillaire, ou un produit coiffant.

Dans un mode de réalisation, la composition selon l'invention est choisie dans le groupe constitué par une crème hydratante, une crème solaire et un shampooing solide.

Lorsque la composition est une émulsion, la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine de la cosmétique.

De façon connue, la composition cosmétique selon l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Il est possible d'utiliser dans les compositions de l'invention le au moins un fructo-oligosaccharide à chaîne courte en association avec au moins un autre produit bénéfique pour l'apparence et/ou le confort cutané, notamment au moins un autre produit contribuant à restaurer, maintenir et/ou préserver l'équilibre du microbiote cutané. Dans certains modes de réalisation, le au moins un autre produit bénéfique pour l'apparence et/ou le confort cutané est choisi dans le groupe constitué par les probiotiques, les dextrines résistantes et les autres fibres prébiotiques.

La composition cosmétique selon l'invention comprend, en association avec le au moins un fructo-oligosaccharide à chaîne courte, au moins un amidon natif.

Le au moins un amidon natif peut être présent dans la composition cosmétique selon l'invention en toute quantité adaptée. Le au moins un amidon natif peut être présent dans la composition cosmétique selon l'invention en une quantité de 0,5 à 20% en poids par rapport au poids de total la composition, en particulier de 4,5 à 15% en poids par rapport au poids total de la composition. Dans le cas où la composition selon l'invention est sous une forme de crème, le au moins un amidon natif est présent de préférence en une quantité de 1 à 10% en poids par rapport au poids total de la composition. Dans certains modes de réalisation, le au moins un amidon natif est présent en une quantité de 4,5%, de 5% ou de 7% en poids par rapport au poids total de la composition. Dans le cas où la composition selon l'invention est sous une forme de solide, le au moins un amidon natif est présent de préférence en une quantité de 10 à 15% en poids par rapport au poids total de la composition. Dans certains modes de réalisation, le au moins un amidon natif est présent en une quantité de 10% ou de 15% en poids par rapport au poids total de la composition.

Dans un mode de réalisation la composition cosmétique selon l'invention comprend :
- au moins 0,5%, en particulier de 0,5 à 5%, en poids par rapport au poids total de la composition, d'au moins un fructo-oligosaccharide à chaîne courte, et
- de 0,5 à 20%, en particulier de 4,5 à 15%, en poids par rapport au poids total de la composition, d'au moins un amidon natif, de préférence d'au moins un amidon natif de maïs.

La présente demande vise ainsi également des compositions cosmétiques comprenant au moins un amidon natif. Préférentiellement, l'amidon natif est issu d'une source d'amidon choisie parmi le maïs, le tapioca, le blé, l'orge, la pomme de terre ou la banane. Préférentiellement, l'amidon natif est de l'amidon natif de maïs. Plus préférentiellement, l'amidon natif est un produit commercialisé sous le nom de marque Meritena^{®} par l'entreprise TEREOS^{®}, encore plus préférentiellement il s'agit du produit commercialisé sous le nom de marque Meritena^{®} 100 par l'entreprise TEREOS^{®}. Les Demanderesses ont en effet démontré que l'inclusion d'amidons natifs dans des compositions cosmétiques selon l'invention permet de conserver, voire d'améliorer, les propriétés sensorielles et physiques des compositions cosmétiques. En particulier, l'association d'au moins un amidon natif avec au moins un fructo-oligosaccharide à chaîne courte dans les compositions cosmétiques selon l'invention permet d'obtenir des compositions cosmétiques, notamment sous forme de crèmes hydratantes, crèmes solaires et/ou shampooings solides, dont les propriétés physiques et sensorielles sont améliorées par rapport à celles des compositions ne comprenant pas une telle association et qui permettent d'améliorer l'apparence et/ou le confort cutané.

Dans certains modes de réalisation, le au moins un fructo-oligosaccharide à chaîne courte est le seul composé de la composition cosmétique contribuant à restaurer, maintenir et/ou préserver l'équilibre du microbiote cutané, en particulier le au moins un fructo-oligosaccharide à chaîne courte est le seul composé de la composition cosmétique améliorant l'apparence et/ou le confort cutané.

### BRÈVE DESCRIPTION DES FIGURES

**Figure 1** est un graphique illustrant la croissance de S. epidermidis en présence de diverses quantités de scFOS à 0h, 8h et 24h.
**Figure 2** est un graphique illustrant la croissance de C. *acnes* en présence de diverses quantités de scFOS à 0h, 8h et 24h.
**Figure 3** est un graphique illustrant la croissance de S. *aureus* en présence de diverses quantités de scFOS à 0h, 8h et 24h.
**Figure 4** est un graphique illustrant la croissance de S. *epidermidis* en présence de diverses quantités de scFOS lorsqu'il est en compétition avec C. *acnes* à 0h, 8h et 24h.

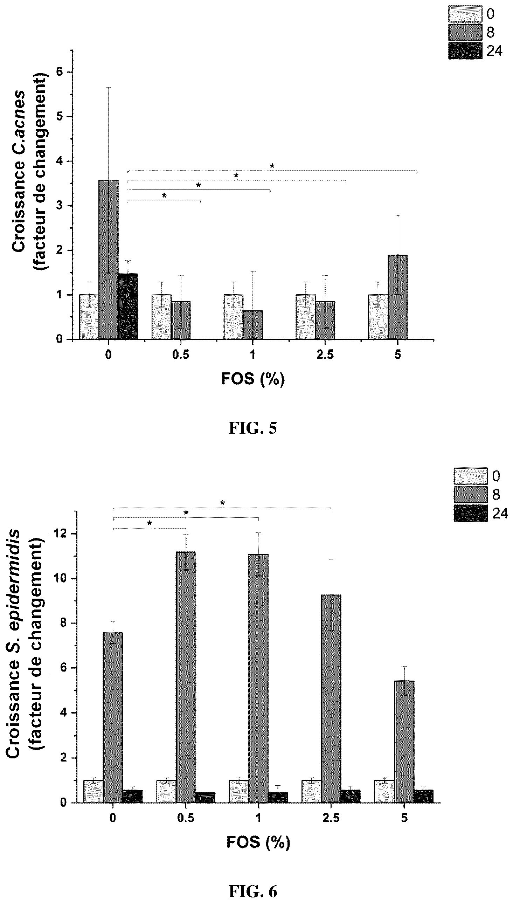

**Figure 5** est un graphique illustrant la croissance de C. *acnes* en présence de diverses quantités de scFOS lorsqu'il est en compétition avec S. *epidermidis* à 0h, 8h et 24h.
**Figure 6** est un graphique illustrant la croissance de S. *epidermidis* en présence de diverses quantités de scFOS lorsqu'il est en compétition avec S. *aureus* à 0h, 8h et 24h.
**Figure 7** est un graphique illustrant la croissance de S. *aureus* en présence de diverses quantités de scFOS lorsqu'il est en compétition avec S. *epidermidis* à 0h, 8h et 24h.
**Figure 8** est un graphique illustrant la force (en Pa) à appliquer pour briser la crème, qui traduit sa viscosité, et la déformation (en %) des crèmes hydratantes selon l'invention. Les histogrammes correspondent à la déformation, tandis que la courbe correspond à la force de viscosité.
**Figure 9** est un graphique illustrant la viscosité en mPa.s des crèmes hydratantes selon l'invention lors de leur étalement à 100 s-1 à 25°C.
**Figure 10** est un histogramme illustrant la fermeté et l'aspect collant, en grammes, des crèmes hydratantes selon l'invention.
**Figure 11** est un graphique illustrant la force (en Pa) à appliquer pour briser la crème, qui traduit sa viscosité, et la déformation (en %) des crèmes solaires selon l'invention. Les histogrammes correspondent à la déformation, tandis que la courbe correspond à la force de viscosité.
**Figure 12** est un graphique illustrant la viscosité en mPa.s des crèmes solaires selon l'invention lors de leur étalement à 100 s-1 à 25°C.
**Figure 13** est un histogramme présentant les valeurs d'humidité (en %) des shampooings solides selon l'invention après 48h à 105°C, pour différentes quantités de fructo-oligosaccharides à chaîne courte. La référence (Réf.) ne comprend pas de fructo-oligosaccharides à chaîne courte.
**Figure 14** est un histogramme présentant les résultats du test de dissolution des shampooings solides selon l'invention après 10 minutes d'agitation à 250 tours par minute à 38°C, pour différentes quantités de fructo-oligosaccharides à chaîne courte. La référence (Réf.) ne comprend pas de fructo-oligosaccharides à chaîne courte.

### EXEMPLES

La présente invention se comprendra mieux à la lecture des exemples suivants qui illustrent non-limitativement l'invention.

### Exemple 1 : Etude de l'effet des scFOS - ACTILIGHT^{®} - sur différentes souches bactériennes

La présente étude a évalué les effets de fructo-oligosaccharides à courte chaîne (scFOS, Actilight^{®} P95, Beghin-Meiji) sur la croissance in vitro et l'activité compétitive de 3 souches bactériennes *(Staphyloccocus epidermidis, Cutibacterium acnes* et *Staphylococcus aureus)* représentatives du microbiote cutané humain.

### Matériel et méthode

### Souches bactériennes

*Staphylococcus epidermidis* (ATCC^{®} 12228) et *Staphylococcus aureus* (ATCC^{®} 6538) ont été acquis de l'American Type Culture Collection (ATCC), tandis que *Cutibacterium acnes* (CCUG 1794T) a été acquis de la Culture Collection University of Gothenburg (CCGU). Les souches bactériennes ont été fournies dans un format lyophilisé et réactivées selon les indications de la banque de cellules respective. En bref, le culot entier a été remis en suspension avec 0,5 ml d'un milieu de croissance approprié (bouillon de soja tryptique (TSB) pour les souches de *Staphylococcus* et TSB + 5% de sang de mouton défibriné pour C. *acnes* (BTSB)). Une fois réhydratées, les suspensions obtenues ont été inoculées dans des tubes stériles contenant le milieu de croissance spécifique de la souche et, après mélange, incubées dans les conditions appropriées (soit 24h à 37 ° C en condition aérobie pour les souches de *Staphylococcus* et 48h à 37 ° C en condition anaérobie pour C. *acnes).* Après 24 heures, les suspensions de souches de *Staphylococcus* ont été striées sur une plaque de gélose au sel de mannitol sélectif pour les staphylocoques, tandis que la suspension de *C*. *acnes* a été striée sur des plaques d'agar pour les bactéries exigeantes après 48h d'incubation. Après 24h et 48h d'incubation dans des conditions appropriées, une seule colonie pour chaque souche bactérienne, prélevée avec une boucle d'inoculation stérile, a été inoculée dans du milieu liquide TSB et BTSB. Lorsque les trois souches bactériennes ont atteint leur phase de croissance mi-log (24h pour les souches de *Staphylococcus* et 48h pour *C*. *acnes),* la concentration de bactéries (charge bactérienne), en unité formant colonie par mL (UFC / mL), présente dans les inoculums, a été déterminée par densitométrie, suivie de comptages CFU après étalement sur gélose de dilutions bactériennes en série. Des inoculums de souches bactériennes fraîches ont été préparés avant chaque expérience pour assurer la cohérence du traitement.

### Préparation de Actilight^{®}

Actilight^{®} a été préparé conformément à Rossi et al., 2005 (10). En bref, en fonction du milieu liquide dans lequel les expériences ont été effectuées, le scFOS a été pesé et dissous à la fois dans le TSB et le milieu minimal (0,9% NaCl dans de l'eau stérile + 0,003% de bouillon de phosphate tryptique (TPB) 1) à une concentration de 20% (p / v). Une fois que le scFOS a été complètement dissous, la stérilisation du milieu a été réalisée par autoclave (121 ° C pendant 20 min).

### Activité bactériostatique et bactéricide des scFOS

La capacité des scFOS à inhiber la croissance des souches bactériennes et / ou à les tuer a été évaluée avec un protocole standard pour la définition de la concentration minimale inhibitrice (CMI) et de la concentration bactéricide minimale (MBC). La CMI est définie comme la concentration la plus faible d'un agent qui empêche la croissance visible des bactéries, tandis que la MBC est la concentration la plus faible d'un agent antibactérien nécessaire pour tuer une bactérie sur une période fixe (généralement 8h et 24h). En bref, 10 x 10⁶ UFC / mL de chaque souche bactérienne ont été exposés à une concentration croissante de scFOS (de 0 à 15% (p / v)), en milieu bouillon liquide (TSB pour S. *aureus* et *S*. *epidermidis,* et BTSB pour *C*. *acnes)* pendant 24h à 37 ° C, sous agitation constante en conditions aérobies. L'impact des différentes concentrations de scFOS sur la croissance des souches bactériennes a été suivi avec une approche de plaque étalée. Des aliquots des inoculums ont été collectés à 0, 8 et 24h, dilués en série et ensemencés sur des plaques d'agar spécifiques (plaques d'agar au sel de mannitol pour les souches de *Staphylococcus* et gélose au sang pour C. *acnes).* Après 24h *(S. aureus* et S. *epidermidis*) et 48h (*C*. *acnes),* les colonies visibles, formées sur les plaques étalées, ont été comptées. Une fois corrigée pour le facteur de dilution approprié, la croissance bactérienne a été calculée comme un facteur de changement par rapport au témoin (t = 0h).

### Impact des scFOS sur la cinétique de croissance des souches bactériennes

La capacité des souches bactériennes testées à utiliser scFOS comme source d'énergie, pour soutenir leur croissance, a été explorée en les exposant à différentes concentrations de scFOS dans un milieu liquide minimal. Brièvement, suite à une centrifugation et un lavage approfondi pour éliminer le milieu de croissance dans lequel elles ont été inoculées, les 3 bactéries (10 x 10⁶ CFU / mL) ont été exposées à des concentrations croissantes de scFOS (de 0 à 15%) dans un milieu minimal à 37 ° C pendant 24h, sous agitation constante et dans des conditions aérobies. Comme décrit ci-dessus, l'impact des différentes concentrations de scFOS sur la croissance des souches bactériennes a été évalué avec une approche de plaque étalée. Des aliquots des inoculums ont été collectés à 8 et 24h, dilués en série et ensemencés sur des plaques d'agar spécifiques (plaques d'agar au sel de mannitol pour les souches de *Staphylococcus* et gélose au sang pour *C*. *acnes).* Après 48h à 37 ° C dans des conditions aérobies, les colonies formées sur les différentes plaques étalées ont été comptées. Une fois corrigée du facteur de dilution approprié, la croissance bactérienne des 3 souches testées a été calculée comme un facteur de changement par rapport à la charge bactérienne de l'inoculum initial (t = 0h).

### Compétition entre les souches de bactéries pour scFOS

La capacité des souches bactériennes à rivaliser pour les scFOS comme source d'énergie a été évaluée au travers des comparaisons *S*. *epidermidis vs C. acnes* et S. *epidermidis vs S. aureus.* Ces compétitions ont été réalisées à différentes concentrations de scFOS (de 0 à 5%) dans un milieu minimal. L'évolution de la compétition a été évaluée avec une approche par plaques étalées, basée sur un moyen sélectif et un comptage différentiel des colonies. En bref, après centrifugation et lavage approfondi pour éliminer le milieu de croissance, 10 x 10⁶ CFU / mL de chaque souche concurrente ont été exposés à des concentrations croissantes de scFOS (de 0 à 5%) dans un milieu minimal (0,9% NaCl dans de l'eau stérile + 0,003% de TPB) à 37 ° C pendant 48h, sous agitation constante et dans des conditions aérobies. Des aliquotes des inoculums ont été prélevés à 0 (charge bactérienne initiale), 4, 8, 24 et 48 h, dilués en série et ensemencés sur des plaques d'agar spécifiques. Pour la compétition *S*. *epidermidis vs S. aureus,* l'ensemencement a été réalisé sur de la gélose au sel de mannitol phénol rouge (MSARP).

Pour la compétition *S*. *epidermidis vs C. acnes,* une stratégie de comptage différentiel des colonies a été appliquée. En bref, les mêmes aliquotes, collectées à des moments différents, ont été ensemencées sur des plaques MSARP et de gélose au sang. Le nombre de CFU / mL de C. *acnes* a été calculé en soustrayant le nombre de colonies obtenu à partir des plaques MSARP, spécifiques pour *S*. *epidermidis,* à celle de la gélose au sang.

Une fois corrigée du facteur de dilution approprié, la croissance des souches bactériennes concurrentes a été calculée comme un facteur de variation par rapport à la charge bactérienne de l'inoculum initial (t = 0h).

### Analyses statistiques

Toutes les analyses statistiques ont été effectuées avec le logiciel OriginLab. Pour déterminer si des différences statistiquement significatives entre les traitements étaient présentes, une analyse de test t a été réalisée. Toutes les données sont présentées sous forme de moyenne ± écart-type (ET) de 3 expériences indépendantes. Les différences entre les groupes ont été considérées comme significatives à p <0,05.

### Résultats

### Impact des scFOS sur la croissance bactérienne et la survie

Avant d'étudier l'effet potentiel des scFOS en tant que prébiotique sur leur croissance, leur impact sur la croissance et la survie de S. *epidermidis, C. acnes* et S. *aureus* a été testé. Les expériences ont été réalisées en milieu de croissance bactérienne spécifique de chaque souche bactérienne (TSB pour *S*. *aureus* et *S*. *epidermidis* et BTSB pour *C*. *acnes).*

Comme le montre le tableau 1, aucun effet bactériostatique ou bactéricide du scFOS n'a été observé sur *S*. *epidermidis, C. acnes* et *S*. *aureus,* puisqu'une croissance significative par rapport à la charge bactérienne initiale a été mise en évidence à toutes les concentrations testées de scFOS. Une diminution des populations de *S*. *epidermidis* et de S. *aureus,* entre 8 et 24h à la même concentration a été observée, probablement due à l'épuisement progressif des nutriments, limitant la survie des bactéries. Une réduction significative de la croissance de C. *acnes* et S. *aureus* a été observée aux concentrations de scFOS les plus élevées (10 et 15% de scFOS).

**Tableau 1. Effet bactériostatique ou bactéricide du scFOS sur S. epidermidis, C. acnes et S. aureus.**

| | ***S. epidermidis*** | | | ***C. acnes*** | | | ***S. aureus*** | | |
|---|---|---|---|---|---|---|---|---|---|
| **scFOS (%)** | **0h** | **8h** | **24h** | **0h** | **8h** | **24h** | **0h** | **8h** | **24h** |
| **0** | 1.0 ± 0.1 | 26.7 ± 0.5 | 23.7 ± 6.3 | 1.0 ± 0.1 | 11.7 ± 1.0 | 0.3 ± 0.5 | 1.0 ± 0.2 | 262.9 ± 11.5 | 141.5 ± 30.1 |
| **1** | 1.0 ± 0.1 | 46.6 ± 4.6 | 15.2 ± 0.3 | 1.0 ± 0.1 | 10.5 ± 0.3 | 2.6 ± 0.2 | 1.0 ± 0.2 | 293.2 ± 36.2 | 196.4 ± 25.9 |
| **5** | 1.0 ± 0.1 | 45.2 ± 4.4 | 9.2 ± 0.2 | 1.0 ± 0.1 | 9.6 ± 1.3 | 1.0 ± 0.5 | 1.0 ± 0.2 | 227.6 ± 35.0 | 154.3 ± 32.5 |
| **10** | 1.0 ± 0.1 | 54.1 ± 9.3 | 3.4 ± 0.2 | 1.0 ± 0.1 | 2.9 ± 0.7 | 2.9 ± 2.2 | 1.0 ± 0.2 | 116.3 ± 22.3 | 82.7 ± 9.6 |
| **15** | 1.0 ± 0.1 | 63.5 ± 19.0 | 3.4 ± 0.6 | 1.0 ± 0.1 | 1.6 ± 0.1 | 0.3 ± 0.1 | 1.0 ± 0.2 | 130.4 ± 4.8 | 28.1 ± 19.3 |

### Activité prébiotique du scFOS sur des souches bactériennes

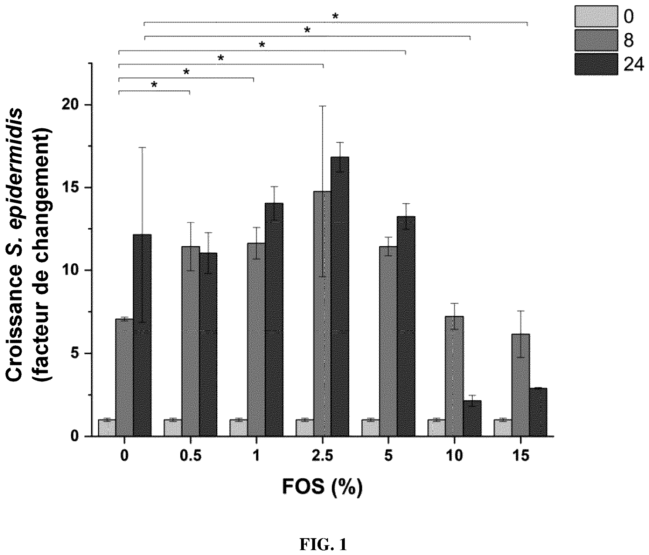

Dans un milieu minimal, la croissance des bactéries est significativement limitée en l'absence de scFOS (0%), pour garantir que la croissance bactérienne dépendait directement de la métabolisation de scFOS. Comme indiqué dans la figure 1, S. *epidermidis* a pu exploiter scFOS comme source d'énergie. En effet, sa charge bactérienne a augmenté significativement après 8h de 0,5 à 5% de scFOS et a eu tendance à augmenter jusqu'à 24h, en présence de 1 et 5% de scFOS. Cependant, des concentrations plus élevées de scFOS (10 et 15%) ont affecté négativement sa croissance par rapport au témoin (0% scFOS).

À l'inverse de S. *epidermidis, S. aureus* et C. *acnes* n'ont pas été en mesure d'exploiter le scFOS comme source d'énergie car la croissance de ces souches bactériennes a toujours été significativement plus faible que celle en absence de scFOS, indépendamment des concentrations et du temps testés. Cependant, alors que la population de *S*. *aureus* était encore capable d'augmenter dans ces conditions, la croissance de la population de *C*. *acnes* a été complètement arrêtée. Une diminution significative de la population des deux bactéries de 8 à 24h a été observée, probablement en raison de l'épuisement progressif des quelques nutriments disponibles dans le milieu minimal qui, à son tour, a conduit à la mort des bactéries (figure 1).

### Concurrence des souches bactériennes pour les scFOS en milieu minimal

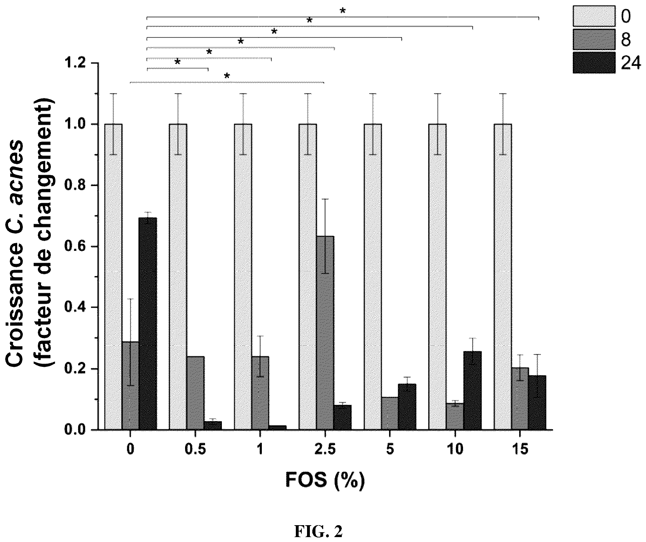

La compétition pour scFOS entre S. *epidermidis* et *C*. *acnes* en milieu minimal a confirmé les résultats observés dans la section précédente. En effet, alors qu'aucun effet positif de scFOS sur la croissance de la population de *C*. *acnes* n'a été mis en évidence à 8h, la présence des scFOS a stimulé la croissance de *S*. *epidermidis* à toutes les doses testées jusqu'à 5% de scFOS (figure 2). Il en résulte un rapport de croissance positif de S. *epidermidis* / *C. acnes* à 8h, avec un maximum atteint à la dose de 1% scFOS (tableau 2).

**Tableau 2. Rapports de croissance S. epidermidis / C. acnes et S. epidermidis / S. aureus.**

| | ***S. epidermidis* / *C. acnes*** | | ***S. epidermidis* / *S. aureus*** | |
|---|---|---|---|---|
| | **Rapport de croissance** | | **Rapport de croissance** | |
| **scFOS (%)** | **8h** | **24h** | **8h** | **24h** |
| **0** | 1.1 | - | 1.6 | 2.0 |
| **0.5** | 7.8 | - | 2.8 | 5.0 |
| **1** | 12.8 | - | 1.8 | - |
| **2.5** | 8.5 | - | 2.5 | 6.0 |
| **5** | 3.4 | - | 1.6 | 2.0 |

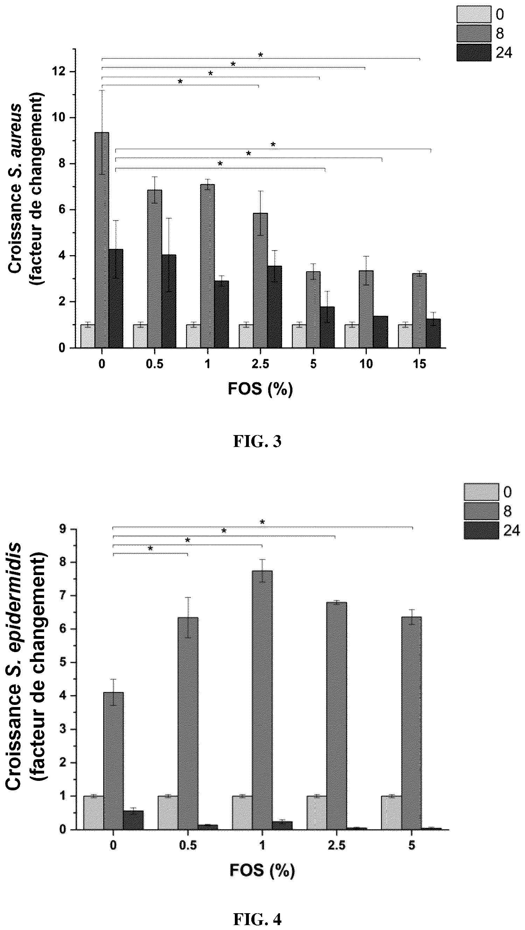

La compétition entre *S*. *epidermidis* et *S*. *aureus* a été caractérisée par des résultats similaires à ceux de la compétition testée précédemment, sans effet positif sur la croissance de S. *aureus* tandis que la croissance de *S*. *epidermidis* a été stimulée par un apport de scFOS à une dose comprise entre 0,5 et 2,5% après 8h de culture en milieu minimal (figure 3). Le rapport calculé entre la croissance de *S*. *epidermidis* et *S*. *aureus a* confirmé la capacité de S. *epidermidis* à croître en utilisant les scFOS au détriment de la croissance de S. *aureus,* avec la stimulation la plus élevée obtenue pour la plus petite dose de scFOS (0,5%) (Tableau 2).

### Exemple 2 : Composition de crème hydratante selon l'invention

Une composition de crème hydratante selon l'invention est décrite dans le tableau 3. Trois alternatives de cette composition ont été préparées, selon que de l'amidon natif de maïs, de blé ou de pomme de terre a été incorporé.

**Tableau 3. Composition de crème hydratante selon l'invention.**

| Phase | Ingrédients | % dans la formulation |
|---|---|---|
| A | Glycérine | 5,0 |
| (aqueuse) | Amidon natif | 5,0 (maïs) ou |
| | | 7,0 (blé) ou |
| | | 7,0 (pomme de terre) |
| | Actilight ^{®} 950 P | 1,0 |
| | Eau | QSP 100 |
| B (lipidique) | Palmitate d'isopropyle | 5,0 |
| | Alcool cétéarylique | 4,0 |
| | Beurre de karité | 3,0 |
| | Huile végétale | 2,0 |
| | Palmitate de cétyle | 1,0 |
| | Vitamine E | 0,1 |
| C (conservateur) | Phénoxyéthanol | 0,2 |

Les pourcentages correspondent à des pourcentages en poids par rapport au poids de la composition.

La composition selon le tableau 3 a été préparée par le procédé suivant :
- Pesée des phases A, B et C,
- Chauffage de la phase A à 85°C pendant une durée allant jusqu'à 45 minutes sous agitation continue,
- Chauffage de la phase B à 75°C,
- Incorporation lente de la phase C, puis de la phase B, dans la phase A en maintenant l'agitation,
- Agitation jusqu'à ce que l'émulsion soit stabilisée.

Une crème blanche avec une texture brillante a été obtenue.

### Exemple 3 : Composition de crème solaire selon l'invention

Une composition de crème solaire selon l'invention est décrite dans le tableau 4.

**Tableau 4. Composition de crème solaire selon l'invention.**

| Phase | Ingrédients | % dans la formulation |
|---|---|---|
| A (aqueuse) | Oxyde de zinc micronisé | 24,4 |
| | Glycérine | 5,0 |
| | Hydrolat de calendula | 2,0 |
| | Amidon de maïs | 4,5 |
| | Actilight ^{®} 950 P | 1,0 |
| | Eau | 47,8 |
| B (lipidique) | Palmitate d'isopropyle | 7,0 |
| | Alcool cétéarylique | 4,0 |
| | Beurre de karité | 2,0 |
| | Huile végétale | 2,0 |
| | Cire d'abeille | 2,0 |
| | Vitamine E | 0,1 |
| C (conservateur) | Phénoxyéthanol | 0,2 |

Les pourcentages correspondent à des pourcentages en poids par rapport au poids de la composition.

La composition selon le tableau 4 a été préparée par le procédé suivant :
- Pesée des phases A, B et C,
- Chauffage de la phase A à 85°C,
- Chauffage de la phase B à 75-80°C,
- Incorporation lente de la phase C, puis de la phase B, dans la phase A en maintenant l'agitation,
- Agitation jusqu'à ce que l'émulsion soit stabilisée.

### Exemple 4 : Composition de shampooing solide selon l'invention

Une composition de shampooing solide selon l'invention est décrite dans le tableau 5.

**Tableau 5. Composition de shampooing solide selon l'invention.**

| Phase | Ingrédients | % dans la formulation |
|---|---|---|
| A (aqueuse) | Sodium cocoyl iséthionate | 50,0 |
| | eau | 21,0 |
| | Amidon de maïs | 15,0 |
| | Actilight ^{®} 950 P | 1,0 |
| | Hydrolat d'ylang-ylang | 4,0 |
| | Hydrolat de rose de Damas | 4,0 |
| B (lipidique) | Beurre de karité | 3,0 |
| | Huile de jojoba | 2,0 |

Les pourcentages correspondent à des pourcentages en poids par rapport au poids de la composition.

La composition selon le tableau 5 a été préparée par le procédé suivant :
- Pesée de la phase B et chauffage à 65°C,
- Mélange doux de l'eau et du surfactant (sodium cocoyl iséthionate) dans un bol avec une spatule,
- Ajout de l'amidon natif de maïs lorsque la pâte est lisse,
- Ajout de la phase B lorsque la pâte est homogène,
- Mélange jusqu'à ce que la pâte soit lisse, puis versage dans des moules
- Séchage des barreaux pendant un jour dans les moules, puis pendant 2 jours hors des moules à température ambiante.

### Exemple 5 : Caractérisation des compositions selon l'invention A-Caractérisation sensorielle

La caractérisation sensorielle des compositions selon l'invention a été réalisée par des groupes de 7 à 8 personnes. 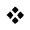

### Caractérisation sensorielle des crèmes hydratantes selon l'invention

La composition des crèmes hydratantes évaluées correspond à celle de la crème hydratante du tableau 3, dans laquelle on a fait varier la quantité de Actilight ^{®} 950 P de 0,5% à 5%.

Le tableau 6 résume les caractéristiques des crèmes hydratantes selon l'invention. Les valeurs indiquées sont des moyennes sur la totalité de l'effectif. La composition de référence est une composition comprenant tous les autres ingrédients, dont 5% d'amidon natif de maïs, mais ne comprenant pas d'Actilight ^{®} 950 P.

**Tableau 6. Caractéristiques des crèmes hydratantes selon l'invention.**

| Quantité de Actilight ^{®} 950 P | Intégrité | Brillance | Fermeté | Epaisseur |
|---|---|---|---|---|
| Référence | 3,95 | 2,8 | 2,82 | 1,63 |
| 0,5% | 3,02 | 2,95 | 1,56 | 1,54 |
| 1,0% | 4,46 | 2,09 | 3,01 | 2,77 |
| 2,5% | 3,61 | 3,7 | 1,52 | 1,42 |
| 5,0% | 4,04 | 3,75 | 1,96 | 1,96 |

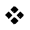 *Caractérisation sensorielle des crèmes solaires selon l'invention*

La composition des crèmes solaires évaluées correspond à celle de la crème solaire du tableau 4, dans laquelle on a fait varier la quantité de Actilight ^{®} 950 P de 0,5% à 5%.

Le tableau 7 résume les caractéristiques des crèmes solaires selon l'invention. Les valeurs indiquées sont des moyennes sur la totalité de l'effectif. La composition de référence est une composition comprenant tous les autres ingrédients, mais ne comprenant pas d'Actilight ^{®} 950 P.

**Tableau 7. Caractéristiques des crèmes solaires selon l'invention.**

| Quantité de Actilight ^{®} 950 P | Intégrité |
|---|---|
| Référence | 2,76 |
| 0,5% | 3,31 |
| 1,0% | 4,02 |
| 2,5% | 3,91 |
| 5,0% | 3,83 |

Les crèmes solaires selon l'invention sont plus compactes que celles sans inclusion de fructo-oligosaccharides à chaîne courte.

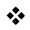 *Caractérisation sensorielle des shampooings solides selon l'invention*

Le tableau 8 résume les caractéristiques des shampooings solides selon l'invention. Le shampooing solide selon l'invention comprend 5% en poids de fructo-oligosaccharides à chaîne courte, et de l'amidon de maïs. La composition de référence est une composition comprenant tous les autres ingrédients, mais ne comprenant pas d'Actilight ^{®} 950 P. La seconde référence est une composition de shampooing solide commercial.

**Tableau 8. Caractéristiques des shampooings solides selon l'invention.**

| | Référence | Référence commerciale | Shampooing solide selon l'invention |
|---|---|---|---|
| Appréciation | 4 | 4,5 | 5 |
| Douceur pendant l'application | 3,5 | 4,5 | 5 |
| Douceur après l'application | 4,5 | 4,5 | 5 |
| Capacité de moussage | 4 | 4 | 4 |
| Stabilité de la mousse | 5 | 5 | 5 |
| Irritation des yeux et des coupures de la peau | 1 | 3 | 1 |

Les shampooings solides selon l'invention ont démontré des propriétés particulièrement intéressantes en termes de ressenti sensoriel pendant et après leur utilisation. En particulier, leur douceur pendant et après application a été remarquée positivement, ainsi que leur capacité de moussage.

**B-Caractérisation physique**

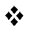 *Caractérisation physique des crèmes hydratantes selon l'invention*

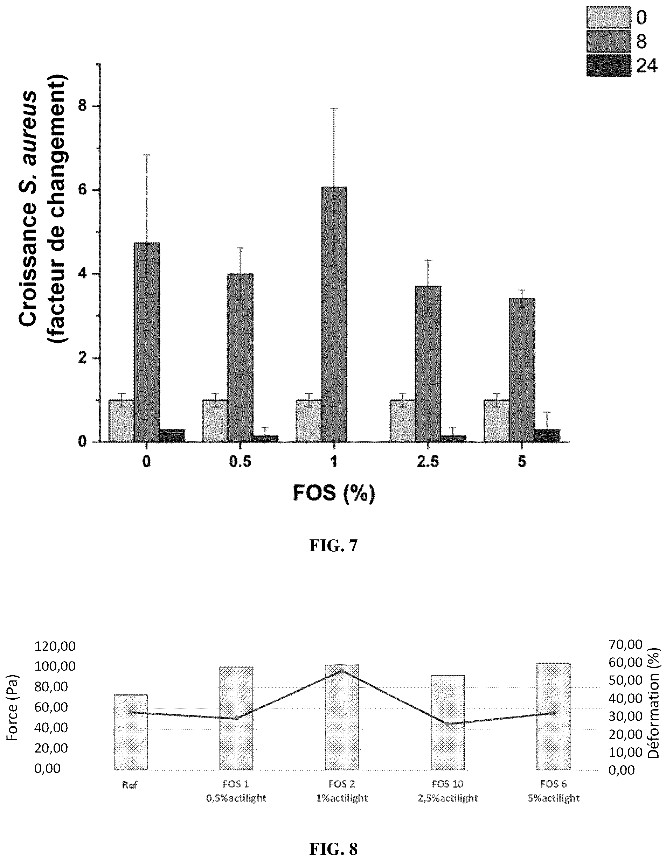

La figure 8 illustre la rhéologie des crèmes hydratantes selon l'invention. Les compositions hydratantes selon l'invention présentent une plus grande élasticité (déformation plus importante) que la référence qui ne comprend pas de fructo-oligosaccharide à chaîne courte. En outre, les compositions selon l'invention présentent une ténacité du même ordre de grandeur que celle de la composition de référence, à l'exception de la composition comprenant 1% de Actilight ^{®} 950 P qui présente une ténacité nettement supérieure.

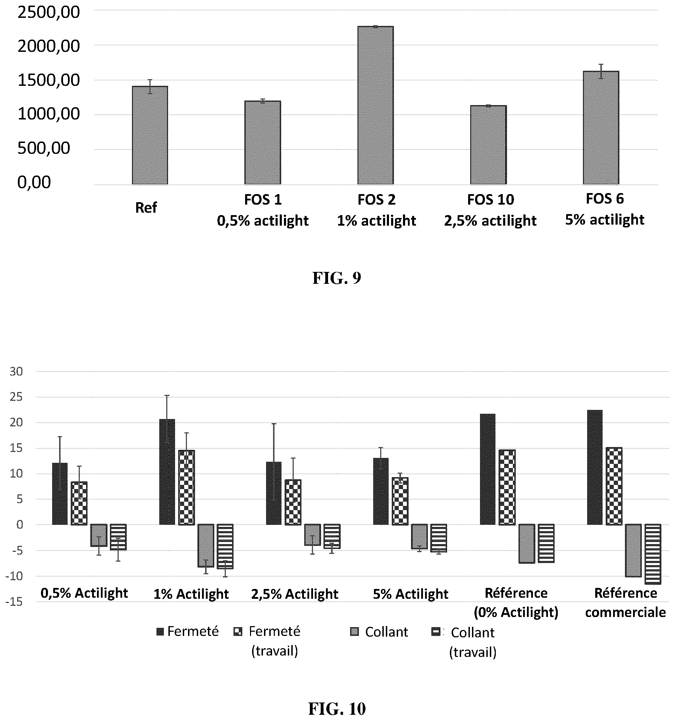

La figure 9 illustre la viscosité lors de l'étalement à 100 s⁻¹ des crèmes hydratantes selon l'invention. Les crèmes hydratantes selon l'invention ont une viscosité du même ordre de grandeur que celle de la composition de référence, à l'exception de la composition comprenant 1% de Actilight ^{®} 950 P qui présente une viscosité nettement supérieure.

L'effet de l'incorporation des fructo-oligosaccharides à chaîne courte sur la couleur des compositions a également été mesuré. Le tableau 9 fournit les valeurs des paramètres L*, a* et b* des crèmes hydratantes selon l'invention, et de deux crèmes de référence : la crème ne comprenant pas de fructo-oligosaccharide à chaîne courte, et une crème de référence commerciale.

Les paramètres L*, a* et b* sont les paramètres du système bien connu L*a*b*, et sont :
- la clarté L* qui prend des valeurs entre 0 (noir) à 100 (blanc de référence) ;
- le paramètre a* qui représente la valeur sur un axe vert → rouge ; et
- le paramètre b* qui représente la valeur sur un axe bleu → jaune.

**Tableau 9. Paramètres L*, a* et b* de deux crèmes de référence et des crèmes hydratantes selon l'invention.**

| Quantité de Actilight ^{®} 950 P | L* | a* | b* |
|---|---|---|---|
| Référence (0%) | 84,87 | -1,53 | 2,92 |
| Référence commerciale | 87,55 | -3,16 | 7,4 |
| 0,5% | 84,37 | -1,76 | 6,17 |
| 1,0% | 86,76 | -1,58 | 5,9 |
| 2,5% | 83,52 | -1,7 | 5,74 |
| 5,0% | 84,85 | -1,73 | 5,73 |

L'incorporation des fructo-oligosaccharides à chaîne courte a un léger impact sur le paramètre b*, avec un léger jaunissement de la crème. Cependant, les crèmes hydratantes selon l'invention restent moins jaunes que la référence commerciale.

Enfin, la figure 10 illustre la fermeté et l'aspect collant des crèmes hydratantes selon l'invention, par comparaison à ceux de la crème de référence qui ne contient pas de fructo-oligosaccharides à chaîne courte, et à ceux de la crème de référence commerciale.

La fermeté et le collant des crèmes hydratantes selon l'invention sont du même ordre de grandeur que ceux des crèmes de référence, aussi bien la crème de référence qui ne comprend pas de fructo-oligosaccharides à chaîne courte que la crème de référence commerciale.

En conclusion, l'incorporation des fructo-oligosaccharides à chaîne courte dans les crèmes hydratantes selon l'invention n'altère pas les propriétés physiques des crèmes. L'incorporation des fructo-oligosaccharides à chaîne courte en une quantité de 1% semble même apporter une amélioration des propriétés physiques des crèmes hydratantes.

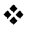 *Caractérisation physique des crèmes solaires selon l'invention*

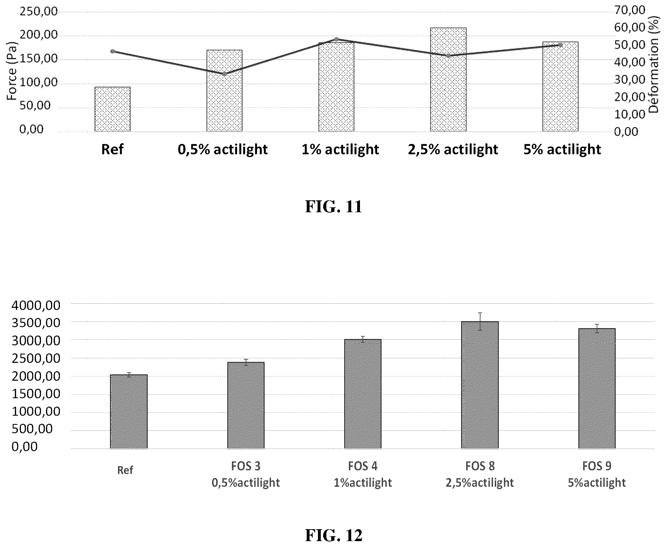

La figure 11 illustre la rhéologie des crèmes solaires selon l'invention. Les compositions solaires selon l'invention présentent une plus grande élasticité (déformation plus importante) que la référence qui ne comprend pas de fructo-oligosaccharide à chaîne courte. En outre, les compositions selon l'invention présentent une ténacité du même ordre de grandeur que celle de la composition de référence.

La figure 12 illustre la viscosité lors de l'étalement à 100 s⁻¹ des crèmes solaires selon l'invention. Les crèmes solaires selon l'invention ont une viscosité qui augmente progressivement avec la proportion de fructo-oligosaccharides à chaîne courte, jusqu'à 2,5%.

L'effet de l'incorporation des fructo-oligosaccharides à chaîne courte sur la couleur des compositions de crèmes solaires a également été mesuré. Le tableau 10 fournit les valeurs des paramètres L*, a* et b* des crèmes solaires selon l'invention, et de trois crèmes solaires de référence : la crème ne comprenant pas de fructo-oligosaccharide à chaîne courte, et deux crèmes solaires de référence commerciales.

**Tableau 10. Paramètres L*, a* et b* de trois crèmes de référence et des crèmes solaires selon l'invention.**

| Quantité de Actilight ^{®} 950 P | L* | a* | b* |
|---|---|---|---|
| Référence (0%) | 92,12 | -0,95 | 3,49 |
| Référence commerciale A | 87,97 | -0,49 | 5,44 |
| Référence commerciale B | 92,52 | -2,52 | 7,29 |
| 0,5% | 92,56 | -0,98 | 3,5 |
| 1,0% | 92,19 | -0,97 | 3,23 |
| 2,5% | 92,41 | -1,01 | 3,22 |
| 5,0% | 91,8 | -0,96 | 3,26 |

L'inclusion des fructo-oligosaccharides à chaîne courte n'a aucun effet significatif sur la couleur des crèmes solaires.

En conclusion, l'inclusion des fructo-oligosaccharides à chaîne courte dans les crèmes solaires permet l'augmentation de leur élasticité et de leur viscosité, sans affecter leur couleur. L'élasticité et la viscosité sont des propriétés souhaitées pour de telles crèmes, et sont souvent atteintes en ajoutant du xanthane et/ou des polymères synthétiques, ce qui n'est pas nécessaire pour les crèmes de l'invention.

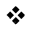 *Caractérisation physique des shampooings solides selon l'invention*

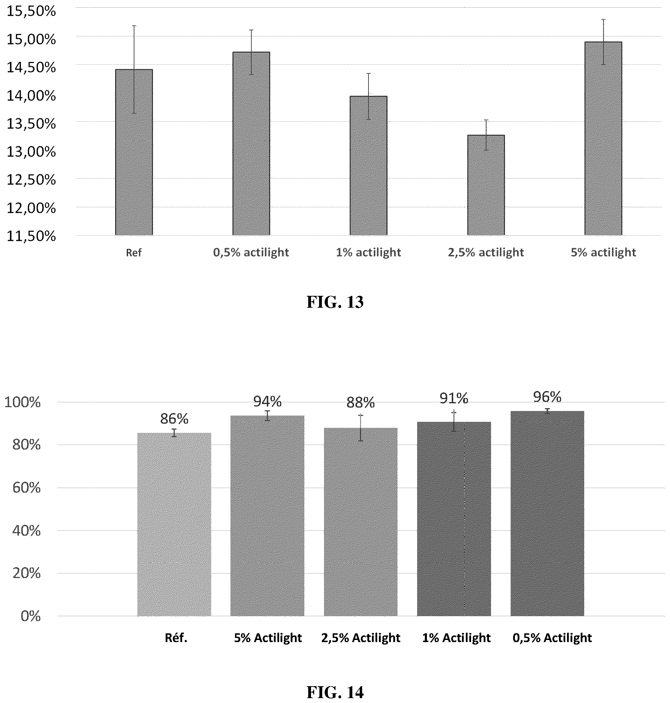

La figure 13 présente les valeurs d'humidité en % des shampooings solides selon l'invention. Les valeurs d'humidité cibles pour un shampooing solide sont la gamme de 10% à 20% d'humidité. Tous les shampooings solides selon l'invention sont donc dans la gamme cible de valeurs d'humidité, quelle que soit la quantité de fructo-oligosaccharides à chaîne courte.

La figure 14 présente les résultats du test de dissolution des shampooings solides selon l'invention. L'inclusion des fructo-oligosaccharides à chaîne courte dans les shampooings solides semble légèrement améliorer la dissolution des shampooings.

L'effet de l'incorporation des fructo-oligosaccharides à chaîne courte sur la couleur des shampooings solides a également été mesuré. Le tableau 11 fournit les valeurs des paramètres L*, a* et b* des shampooings solides selon l'invention, et de trois shampooings solides de référence : le shampooing solide ne comprenant pas de fructo-oligosaccharide à chaîne courte, et deux shampooings solides de référence commerciaux A et B.

**Tableau 11. Paramètres L*, a* et b* de trois shampooings solides de référence et des shampooings solides selon l'invention.**

| Quantité de Actilight ^{®} 950 P | L* | a* | b* |
|---|---|---|---|
| Référence (0%) | 78,54 | -1,31 | 1,78 |
| Référence commerciale A | 95,8 | -1,58 | 7,67 |
| Référence commerciale B | 71,73 | -1,03 | -0,06 |
| 0,5% | 82,51 | -1,8 | 11,65 |
| 1,0% | 81,38 | -1,71 | 12,03 |
| 2,5% | 76,21 | -2,61 | 10,47 |
| 5,0% | 82,14 | -1,94 | 10,38 |

L'inclusion des fructo-oligosaccharides à chaîne courte n'a aucun effet significatif sur la couleur des shampooings solides.

En conclusion, les shampooings solides selon l'invention présentent des valeurs d'humidité adaptées, ont une dissolution légèrement améliorée, et leur couleur n'est pas dégradée par rapport aux shampooings solides ne comprenant pas de fructo-oligosaccharide à chaîne courte.

### Exemple 6 : Effets de l'association de scFOS avec de l'amidon natif

La présente analyse évalue les effets de l'association des fructo-oligosaccharides à courte chaîne (scFOS, Actilight^{®} P95, Beghin-Meiji) avec de l'amidon natif de maïs (Meritena^{®} 100, TEREOS^{®}) sur les propriétés de texture ainsi que les propriétés sensorielles des formulations cosmétiques.

Des compositions de crème hydratante selon l'invention et des compositions comparatives sont décrites dans le tableau 12.

**Tableau 12. Composition d'une crème hydratante selon l'invention et de quatre compositions comparatives.**

| Composition | | % dans la formulation | | | | |
|---|---|---|---|---|---|---|
| Phase | Ingrédients | Invention | A | B | C | D |
| A (aqueuse) | Glycérine | 5,0 | 5,0 | 5,0 | 5,0 | 5,0 |
| | Amidon natif | 5,0 | 5,0 | - | | - |
| | Actilight ^{®} 950 P | 5,0 | - | - | 5,0 | 5,0 |
| | Xanthane | - | - | 4,0 | 4,0 | - |
| | Eau | 69,7 | 74,7 | 75,7 | 70,7 | 74,7 |
| B (lipidique) | Palmitate d'isopropyle | 5,0 | 5,0 | 5,0 | 5,0 | 5,0 |
| | Alcool cétéarylique | 4,0 | 4,0 | 4,0 | 4,0 | 4,0 |
| | Beurre de karité | 3,0 | 3,0 | 3,0 | 3,0 | 3,0 |
| | Huile végétale | 2,0 | 2,0 | 2,0 | 2,0 | 2,0 |
| | Palmitate de cétyle | 1,0 | 1,0 | 1,0 | 1,0 | 1,0 |
| | Vitamine E | 0,1 | 0,1 | 0,1 | 0,1 | 0,1 |
| C (conservateur) | phénoxyéthanol | 0,2 | 0,2 | 0,2 | 0,2 | 0,2 |

La composition selon le tableau 12 a été préparée par le procédé suivant :
- Pesée des phases A, B et C,
- Chauffage de la phase A à 85°C,
- Chauffage de la phase B à 75°C,
- Incorporation lente de la phase C, puis de la phase B, dans la phase A en maintenant l'agitation,
- Agitation jusqu'à ce que l'émulsion soit stabilisée.

La quantité de 4% de xanthane a été retenue comme quantité d'épaississant (formulations B et C) pertinente pour comparaison avec 5% d'amidon de maïs (formulation A et formulation selon l'invention), afin de comparer formulations à textures proches (par exemple, 5% de xanthane conduirait à une formulation trop visqueuse).

### Caractérisation rhéologique des compositions

Les propriétés rhéologiques des compositions selon le tableau 12 sont présentées dans le tableau 13.

**Tableau 13. Propriétés rhéologiques des compositions selon l'exemple 6.**

| Composition | Point d'écoulement à 25°C | | Viscosité (mPa.s) lors de l'étalement à 100 s⁻¹ |
|---|---|---|---|
| | Elasticité (déformation %) | Résistance (Force pour casser la crème en Pa) | |
| Invention (scFOS + Amidon) | 60,28 | 55,02 | 1621,17 ± 10,69 |
| A (amidon) | 42.45 | 55,75 | 1401,63 ± 101,04 |
| B (xanthane) | 147,73 | 48,16 | 1288,50 ± 44,27 |
| C (scFOS+ xanthane) | 103,00 | 135,82 | 2228,36 ± 88,58 |
| D (scFOS) | 1,22 | 0,04 | 29,1731 ± 3,13 |

L'association de scFOS avec un agent épaississant a augmenté la viscosité lors de l'étalement. En effet, l'association selon l'invention présente une viscosité lors de l'étalement plus élevée par rapport à celle de la composition A comprenant uniquement de l'amidon. Il en va de même pour l'association de scFOS avec le xanthane.

Cependant, lorsque les scFOS sont associés avec de l'amidon, la résistance de la crème n'est pas impactée. En effet, l'association de scFOS avec de l'amidon natif selon l'invention n'a pas modifié la viscosité résistance à la composition A comprenant uniquement de l'amidon.

### Caractérisation de texture des compositions

Les propriétés de texture par pénétrométrie à 25°C des compositions selon le tableau 12 sont présentées dans le tableau 14.

**Tableau 14. Propriétés de texture des compositions selon le tableau 12.**

| Composition | Fermeté (g) | Fermeté travail (g.s) | Collant (g) | Collant travail (g.s) |
|---|---|---|---|---|
| Invention (scFOS + Amidon) | 9,153 ±0,48 | 13,018 ±1,035 | -4,649 ±0,198 | -5,221 ±0,235 |
| A (amidon) | 14,543 ±1,203 | 21,702 ±3,962 | -7,391 ±0,866 | -7,285 ±0,326 |
| B (xanthane) | 12,476 ±0,309 | 18,679 ±0,73 | -5,79 ±0,562 | -4,339 ±0,602 |
| C (scFOS+ xanthane) | 14,947 ±0,499 | 29,774 ±1,126 | -6,698 ±0,481 | -4,552 ±0,511 |
| D (scFOS) | - | - | - | - |

La présence des scFOS en association avec de l'amidon natif a abaissé la fermeté globale de la formulation, alors que le phénomène inverse a été observé avec l'association des scFOS avec le xanthane.

### Caractérisation sensorielle des compositions

La caractérisation sensorielle des compositions selon le tableau 12 a été réalisée par des groupes de 5 personnes.

Les propriétés sensorielles des compositions selon le tableau 12 sont présentées dans le tableau 15.

**Tableau 15. Propriétés sensorielles des compositions selon le tableau 12.**

| | A (amidon) | Invention (scFOS + Amidon) | B (xanthane) | C (scFOS+ xanthane) |
|---|---|---|---|---|
| Brillance | 3,20 ±0,27 | 4,30 ±0,67 | 4,20 ±0,45 | 2,50 ±0,71 |
| Fermeté | 2,50 ±1,00 | 1,70 ±0,91 | 3,00 ±1,41 | 3,60± 0,89 |
| Epaisseur | 2,10 ±0,89 | 1,20 ±0,27 | 2,10 ±0,74 | 2,80 ±0,84 |
| Effet gras | 3,00 ±1,17 | 1,90 ±0,89 | 3,20 ±1,10 | 3,00 ±1,41 |
| Effet collant | 1,60 ±0,89 | 1,90 ±0,74 | 2,70 ±1,20 | 2,80 ±1,04 |
| Effet glissement | 3,50 ±1,00 | 3,00 ±0,82 | 2,60 ±1,14 | 2,60 ±0,55 |
| Absorption | 3,30 ±0,67 | 3,70 ±0,45 | 2,00 ±0,94 | 2,10 ±1,14 |
| Appréciation | 3,30 ±0,84 | 3,50 ±1,12 | 1,60 ±1,14 | 1,70 ±1,48 |

La formulation comprenant l'association d'Actilight avec de l'amidon était plus appréciée par rapport à la formulation comprenant uniquement de l'amidon grâce à son toucher moins gras et grâce à la meilleure brillance apportée.

La présence d'Actilight n'a pas modifié les caractéristiques de texture des formulations à base d'amidon, mais l'évaluation sensorielle a montré des modifications avantageuses.

La formulation à base d'amidon de maïs et d'Actilight s'est révélée meilleure que les autres formulations, notamment par rapport aux formulations à base de xanthane.

### LISTE DES REFERENCES

1. Sfriso, R.; Egert, M.; Gempeler, M.; Voegeli, R.; Campiche, R. Revealing the secret life of skin - with the microbiome you never walk alone. Int J Cosmet Sci 2020. 42(2): p. 116-126, DOI: 10.1111/ics.12594.
2. Kong, H.H.; Segre, J.A. Skin microbiome: looking back to move forward. J Invest Dermatol 2012. 132(3 Pt 2): p. 933-9, DOI: 10.1038/jid.2011.417.
3. Sanford, J.A.; Gallo, R.L. Functions of the skin microbiota in health and disease. Semin Immunol 2013. 25(5): p. 370-7, DOI: 10.1016/j.smim.2013.09.005.
4. Krutmann, J. Pre- and probiotics for human skin. J Dermatol Sci 2009. 54(1): p. 1-5, DOI: 10.1016/j.jdermsci.2009.01.002.
5. Grice, E.A.; Segre, J.A. The skin microbiome. Nat Rev Microbiol 2011. 9(4): p. 244-53, DOI: 10.1038/nrmicro2537.
6. Ladizinski, B.; McLean, R.; Lee, K.C.; Elpern, D.J.; Eron, L. The human skin microbiome. Int J Dermatol 2014. 53(9): p. 1177-9, DOI: 10.1111/ijd.12609.
7. Al-Ghazzewi, F.H.; Tester, R.F. Impact of prebiotics and probiotics on skin health. Benef Microbes 2014. 5(2): p. 99-107, DOI: 10.3920/BM2013.0040.
8. Dreno, B.; Araviiskaia, E.; Berardesca, E.; Gontijo, G.; Sanchez Viera, M.; Xiang, L.F.; Martin, R.; Bieber, T. Microbiome in healthy skin, update for dermatologists. J Eur Acad Dermatol Venereol 2016. 30(12): p. 2038-2047, DOI: 10.1111/jdv.13965.
9. Grice, E.A.; Kong, H.H.; Renaud, G.; Young, A.C.; Program, N.C.S.; Bouffard, G.G.; Blakesley, R.W.; Wolfsberg, T.G.; Turner, M.L.; Segre, J.A. A diversity profile of the human skin microbiota. Genome Res 2008. 18(7): p. 1043-50, DOI: 10.1101/gr.075549.107.
10. Rossi, M.; Corradini, C.; Amaretti, A.; Nicolini, M.; Pompei, A.; Zanoni, S.; Matteuzzi, D. Fermentation of fructooligosaccharides and inulin by bifidobacteria: a comparative study of pure and fecal cultures. Appl Environ Microbiol 2005. 71(10): p. 6150-8, DOI: 10.1128/AEM.71.10.6150-6158.2005.

## Revendications

1. Méthode cosmétique non-thérapeutique pour équilibrer le microbiote cutané, comprenant une étape d'application sur tout ou partie de la peau, des phanères et/ou des muqueuses d'une composition cosmétique comprenant, dans un support cosmétiquement acceptable, au moins un fructo-oligosaccharide à chaîne courte constitué de 2 à 4 molécules de fructose et contenant une molécule de glucose à l'une des extrémités et au moins un amidon natif.

2. Méthode cosmétique non-thérapeutique selon la revendication 1, pour améliorer l'apparence et/ou le confort cutané.

3. Méthode cosmétique non-thérapeutique selon la revendication 2, dans laquelle améliorer l'apparence
et/ou le confort cutané comprend réduire au moins un désagrément cutané choisi dans le groupe constitué par les rougeurs, les tiraillements, la peau terne et les démangeaisons.

4. Méthode cosmétique non-thérapeutique selon l'une quelconque des revendications 1 à 3, dans laquelle
le au moins un fructo-oligosaccharide est issu de la betterave sucrière.

5. Méthode cosmétique non-thérapeutique selon l'une quelconque des revendications 1 à 4, dans laquelle
le au moins un fructo-oligosaccharide à chaîne courte est un mélange :
- d'un fructo-oligosaccharide contenant une unité de glucose et deux unités de fructose, aussi appelé 1-kestose ou GF2, présentant un degré de polymérisation égal à 3 ;
- d'un fructo-oligosaccharide contenant une unité de glucose et trois unités de fructose, aussi appelé nystose ou GF3, présentant un degré de polymérisation égal à 4 ; et
- d'un fructo-oligosaccharide contenant une unité de glucose et quatre unités de fructose, aussi appelé 1-bêta-fructofuranosylnystose ou GF4, présentant un degré de polymérisation égal à 5.

6. Méthode cosmétique non-thérapeutique selon l'une quelconque des revendications 1 à 5, dans laquelle
le au moins un amidon natif est un amidon natif de maïs.

7. Méthode cosmétique non-thérapeutique selon l'une quelconque des revendications 1 à 6, dans laquelle
l'au moins un amidon natif est compris dans la composition cosmétique en une quantité de 0,5 à 20% en poids par rapport au poids total de la composition cosmétique.

8. Méthode cosmétique non-thérapeutique selon l'une quelconque des revendications 1 à 7, dans laquelle
le au moins un fructo-oligosaccharide à chaîne courte est compris dans la composition cosmétique en une quantité d'au moins 0,5% en poids par rapport au poids total de la composition cosmétique.

9. Utilisation non-thérapeutique d'une composition cosmétique comprenant, dans un support cosmétiquement acceptable, au moins un fructo-oligosaccharide à chaîne courte constitué de 2 à 4 molécules de fructose et contenant une molécule de glucose à l'une des extrémités, et au moins un amidon natif, pour équilibrer le microbiote cutané.

10. Composition cosmétique comprenant, dans un support cosmétiquement acceptable, au moins un fructo-oligosaccharide à chaîne courte constitué de 2 à 4 molécules de fructose et contenant une molécule de glucose à l'une des extrémités et au moins un amidon natif,
dans laquelle le au moins un fructo-oligosaccharide à chaîne courte est un mélange :
- d'un fructo-oligosaccharide contenant une unité de glucose et deux unités de fructose, aussi appelé 1-kestose ou GF2, présentant un degré de polymérisation égal à 3 ;
- d'un fructo-oligosaccharide contenant une unité de glucose et trois unités de fructose, aussi appelé nystose ou GF3, présentant un degré de polymérisation égal à 4 ; et
- d'un fructo-oligosaccharide contenant une unité de glucose et quatre unités de fructose, aussi appelé 1-bêta-fructofuranosylnystose ou GF4, présentant un degré de polymérisation égal à 5.

11. Composition cosmétique selon la revendication **10,** dans laquelle le au moins un amidon natif est un amidon natif de maïs.

12. Composition cosmétique selon la revendication **10** ou **11,** dans laquelle le au moins un amidon natif est compris en une quantité de 0,5 à 20% en poids par rapport au poids total de la composition cosmétique.

13. Composition cosmétique selon l'une quelconque des revendications **10** à **12,** dans laquelle le au moins un amidon natif est compris en une quantité de plus de 5% à 20% en poids par rapport au poids total de la composition cosmétique.

14. Composition cosmétique selon l'une quelconque des revendications **10** à **13,** dans laquelle le au moins un fructo-oligosaccharide à chaîne courte est compris en une quantité d'au moins 0,5% en poids par rapport au poids total de **la composition** cosmétique.

15. Composition cosmétique selon l'une quelconque des revendications **10** à **14,** dans laquelle la composition cosmétique est sous la forme d'un produit dermo-cosmétique, d'un produit capillaire, d'un produit d'hygiène intime, d'un pansement et/ou d'un produit cicatrisant.

## Patentansprüche

1. Nicht-therapeutische kosmetische Methode
zum Ausgleich der Hautmikrobiota, umfassend einen Schritt
zur Anwendung auf die gesamte oder einen Teil der Haut, der Hautanhangsgebilde und/oder Schleimhäute einer kosmetischen Zusammensetzung, die in einem kosmetisch annehmbaren Träger mindestens ein kurzkettiges Fructo-Oligosaccharid umfasst, das aus 2 bis 4 Fruktosemolekülen besteht und an einem der Enden ein Glukosemolekül enthält
und mindestens eine native Stärke.

2. Nicht-therapeutische kosmetische Methode
nach Anspruch **1,** um das Erscheinungsbild und/oder den
Hautkomfort zu verbessern.

3. Nicht-therapeutische kosmetische Methode
nach Anspruch 2, wobei das Verbessern des Erscheinungsbilds und/oder des Hautkomforts die Reduzierung von mindestens einem Hautunbehagen umfasst, das aus der Gruppe ausgewählt wurde, die aus Rötungen, Spannungsgefühlen, stumpfer Haut und Juckreiz besteht.

4. Nicht-therapeutische kosmetische Methode
nach einem der Ansprüche **1** bis **3,** wobei
mindestens ein Fructo-Oligosaccharid aus Zuckerrüben gewonnen wird.

5. Nicht-therapeutische kosmetische Methode
nach einem der Ansprüche **1** bis **4,** wobei
das mindestens eine kurzkettige Fructo-Oligosaccharid eine Mischung ist:
- aus einem Fructo-Oligosaccharid, das eine Glukoseeinheit und zwei Fruktoseeinheiten enthält, auch 1-Kestose oder GF2 genannt, mit einem Polymerisationsgrad von 3;
- aus einem Fructo-Oligosaccharid, das eine Glukoseeinheit und drei Fruktoseeinheiten enthält, auch Nystose oder GF3 genannt, mit einem Polymerisationsgrad von 4; und
- aus einem Fructo-Oligosaccharid, das eine Glukoseeinheit und vier Fruktoseeinheiten enthält, auch als 1-Beta-Fructofuranosylnystose oder GF4 bezeichnet, mit einem Polymerisationsgrad von 5.

6. Nicht-therapeutische kosmetische Methode
nach einem der Ansprüche **1** bis **5,** wobei
die mindestens eine native Stärke eine native Maisstärke ist.

7. Nicht-therapeutische kosmetische Methode
nach einem der Ansprüche **1** bis **6,** wobei
die mindestens eine native Stärke in einer Menge von 0,5 bis 20 Gew.-% bezogen auf das Gesamtgewicht der kosmetischen Zusammensetzung in der kosmetischen Zusammensetzung enthalten ist.

8. Nicht-therapeutische kosmetische Methode
nach einem der Ansprüche **1** bis **7,** wobei
das mindestens ein kurzkettiges Fructo-Oligosaccharid in einer Menge von mindestens 0,5 Gew.-% bezogen auf das Gesamtgewicht der kosmetischen Zusammensetzung in der kosmetischen Zusammensetzung enthalten ist.

9. Nicht-therapeutische Anwendung
einer kosmetischen Zusammensetzung umfassend, in einem
kosmetisch annehmbaren Träger, mindestens ein kurzkettiges Fructo-Oligosaccharid, das aus 2 bis 4 Fruktosemolekülen besteht und an einem der Enden ein Glukosemolekül enthält, und
mindestens eine native Stärke, um die Hautmikrobiota auszugleichen.

10. Kosmetische Zusammensetzung, umfassend, in einem kosmetisch annehmbaren Träger,
mindestens ein kurzkettiges Fructo-Oligosaccharid, das aus 2 bis 4 Fruktosemolekülen besteht und an einem der Enden ein Glukosemolekül und mindestens eine native Stärke enthält,
wobei das mindestens eine kurzkettige Fructo-Oligosaccharid eine Mischung ist:
- aus einem Fructo-Oligosaccharid, das eine Glukoseeinheit und zwei Fruktoseeinheiten enthält, auch 1-Kestose oder GF2 genannt, mit einem Polymerisationsgrad von 3;
- aus einem Fructo-Oligosaccharid, das eine Glukoseeinheit und drei Fruktoseeinheiten enthält, auch Nystose oder GF3 genannt, mit einem Polymerisationsgrad von 4; und
- aus einem Fructo-Oligosaccharid, das eine Glukoseeinheit und vier Fruktoseeinheiten enthält, auch als 1-Beta-Fructofuranosylnystose oder GF4 bezeichnet, mit einem Polymerisationsgrad von 5.

11. Kosmetische Zusammensetzung nach Anspruch **10,** wobei die mindestens eine native Stärke eine native Maisstärke ist.

12. Kosmetische Zusammensetzung nach Anspruch **10** oder **11,** wobei die mindestens eine native Stärke in einer Menge von 0,5 bis 20 Gew.-% bezogen auf das Gesamtgewicht der kosmetischen Zusammensetzung enthalten ist.

13. Kosmetische Zusammensetzung nach einem der Ansprüche **10** bis **12,** wobei die mindestens eine native Stärke in einer Menge von mehr als 5 % bis 20 % Gew.-% bezogen auf das Gesamtgewicht der kosmetischen Zusammensetzung enthalten ist.

14. Kosmetische Zusammensetzung nach einem der Ansprüche **10** bis **13,** wobei das mindestens eine kurzkettige Fructo-Oligosaccharid in einer Menge von mindestens 0,5 Gew.-% bezogen auf das Gesamtgewicht der kosmetischen Zusammensetzung enthalten ist.

15. Kosmetische Zusammensetzung nach einem der Ansprüche **10** bis **14,** wobei die kosmetische Zusammensetzung in Form eines dermokosmetischen Produkts, eines Haarprodukts, eines Intimpflegeprodukts, eines Verbands und/oder eines Wundheilungsprodukts ist.

## Claims

1. Non-therapeutic cosmetic method for balancing the skin microbiota, comprising a step of applying to all or part of the skin, skin appendages and/or mucous membranes a cosmetic composition comprising, in a cosmetically acceptable carrier, at least one fructo-oligosaccharide short chain consisting of 2 to 4 molecules of fructose and containing a molecule of glucose at one of the extremities and at least one native starch.

2. Non-therapeutic cosmetic method according to claim **1,** for improving the skin appearance and/or skin comfort.

3. Non-therapeutic cosmetic method according to claim **2,** wherein improving the skin appearance and/or skin comfort comprises reducing at least one skin discomfort chosen from the group consisting of redness, tightness, dull skin and itching.

4. Non-therapeutic cosmetic method according to any one of claims **1** to **3,** wherein the at least one fructo-oligosaccharide is derived from sugar beet.

5. Non-therapeutic cosmetic method according to any one of claims **1** to **4,** wherein the at least one short-chain fructo-oligosaccharide is a mixture of:
- a fructo-oligosaccharide containing one glucose unit and two fructose units, also called 1-kestose or GF2, with a degree of polymerization equal to 3;
- a fructo-oligosaccharide containing one glucose unit and three fructose units, also called nystose or GF3, with a degree of polymerization equal to 4; and
- a fructo-oligosaccharide containing one glucose unit and four fructose units, also called 1-beta-fructofuranosylnystose or GF4, with a degree of polymerization equal to 5.

6. Non-therapeutic cosmetic method according to any one of claims **1** to **5,** wherein the at least one native starch is a native corn starch.

7. Non-therapeutic cosmetic method according to any one of claims **1** to **6,** wherein the at least one native starch is included in the cosmetic composition in an amount ranging from 0.5% to 20% by weight relative to the total weight of the cosmetic composition.

8. Non-therapeutic cosmetic method according to any one of claims **1** to **7,** wherein the at least one short-chain fructo-oligosaccharide is included in the cosmetic composition in an amount of at least 0.5% by weight relative to the weight total of the cosmetic composition.

9. Non-therapeutic use of a cosmetic composition comprising, in a cosmetically acceptable carrier, at least one short-chain fructo-oligosaccharide consisting of 2 to 4 molecules of fructose and containing a molecule of glucose at one of the extremities, and at least one native starch, to balance the skin microbiota.

10. Cosmetic composition comprising, in a cosmetically acceptable carrier, at least one short-chain fructo-oligosaccharide consisting of 2 to 4 molecules of fructose and containing a molecule of glucose at one of the extremities and at least one native starch, wherein the at least one short-chain fructo-oligosaccharide is a mixture of:
- a fructo-oligosaccharide containing one glucose unit and two fructose units, also called 1-kestose or GF2, with a degree of polymerization equal to 3;
- a fructo-oligosaccharide containing one glucose unit and three fructose units, also called nystose or GF3, with a degree of polymerization equal to 4; and
- a fructo-oligosaccharide containing one glucose unit and four fructose units, also called 1-beta-fructofuranosylnystose or GF4, with a degree of polymerization equal to 5.

11. Cosmetic composition according to claim **10,** wherein the at least one native starch is a native corn starch.

12. Cosmetic composition according to claim **10** or **11,** wherein the at least one native starch is included in an amount ranging from 0.5 to 20% by weight relative to the total weight of the cosmetic composition.

13. Cosmetic composition according to any one of claims **10** to **12,** wherein the at least one native starch is included in an amount of more than 5% to 20% by weight relative to the total weight of the cosmetic composition.

14. Cosmetic composition according to any one of claims **10** to **13,** wherein the at least one short-chain fructo-oligosaccharide is included in an amount of at least 0.5% by weight relative to the total weight of the cosmetic composition.

15. Cosmetic composition according to any one of claims **10** to **14,** wherein the cosmetic composition is in the form of a dermo-cosmetic product, a hair product, an intimate hygiene product, a dressing and/or a healing product.
